# EUROPEAN PATENT APPLICATION

(11) **EP 3 875 583 A1**
(43) Date of publication of application: **08.09.2021**
(21) Application number: 20382163.2
(22) Date of filing: 06.03.2020
(51) Int. Cl.: C12N 9/00, C02F 3/02, C02F 3/34, C12N 9/10, C12N 9/88, C02F 101/34, C02F 101/30

(54) **GENETIC CASSETTE COMPRISING THE PHT PATHWAY GENES, RECOMBINANT HOST CELLS COMPRISING IT AND THEIR USE IN THE DEGRADATION AND VALORIZATION OF PHTHALATES**

(71) Applicant: Consejo Superior de Investigaciones Científicas (CSIC), 28006 Madrid (ES)
(72) Inventor: DÍAZ FERNÁNDEZ, Eduardo, 28040 Madrid (ES); SANZ MATA, David, 28040 Madrid (ES)
(74) Representative: Pons

(57) **Abstract**

The present invention provides a recombinant genetic cassette which comprises the catabolic genes involved in the pht pathway as well as an SBP-dependent secondary transporter that are essential for microbial phthalate degradation. The invention also relates to recombinant host cells comprising this cassette and are useful for the aerobic or anaerobic biodegradation of phthalates, preferably o-phthalate (PAs), and for the bioconversion of PAs towards the biosynthesis of value-added biodegradable polymers, such as polyhydroxyalkanoates (PHAs), preferably polyhydroxybutyrates (PHBs).

SEQUENCE LISTING

<110>  Consejo Superior de Investigaciones Científicas (CSIC)

<120>  GENETIC CASSETTE COMPRISING THE PHT PATHWAY GENES, RECOMBINANT
       HOST CELLS COMPRISING IT AND THEIR USE IN THE DEGRADATION AND
       VALORIZATION OF PHTHALATES

<130>  EP1641.1544

<160>  22

<170>  PatentIn version 3.5

<210>  1
<211>  600
<212>  DNA
<213>  Aromatoleum aromaticum

<400>  1
atgaaaagac tgatagtggg gatcactggg gcgacggggg tggtgttcgg ggttcggttg      60

ctcgaggcgc tgcagggcac cgacgtggag acgcacctgg tgctgagcaa gtgggcgatc     120

cagacgatcg agcacgagac gtcctacacg gccaagcagg tgcgggcgct cgccgcggtc     180

gatcacgtcg aaggcaacat gggcgcgtcg atctcgtcgg gctcgttcgt gaccgaaggg     240

atggtgatcg cgccgtgctc gatgaggagc ctcgcggcga tcgcccacgg cacgggcgac     300

cacctggtgc atcgggcggc cgacgtgatc ctcaaggagc ggcgtcgcct ggtgctcgtg     360

acgcgcgaga tgccgctctc ggacatccat ctcgagaaca tgctcaagct ctcgaagatg     420

ggcgtgacga tcatgccgcc gatgcccgcg ttctataacc acccgcagac ggtcgacgac     480

atcgtggatc acgtcgttgc ccggattctc gatcaattcg gcctgccggc cgagttcgcg     540

cggcgctggg agggggagat gcgcaaccgc aaggttgcca atctccagcc gcagaagtaa      600


<210>  2
<211>  1584
<212>  DNA
<213>  Aromatoleum aromaticum

<400>  2
atgaacgatc tggcaacgaa agaaatcgat gcggtggccc tgcgggccgg ggtcaaggac      60

ctgcgtgaag cgctcgagtg gttccgcacc cagggctacc tggtcgagac cgacaaggaa     120

gtcaatcctg acctcgagat caccggtctg cagaagatct tcgacggcag cctgccgatg     180

ctcttcaaca acgtcaaagg catgccccat gcccgcgcga tcacgaacct ctttggcgac     240

atccgtgtcg tcgaaggcct cttcggctgg gagaactcgc tcgaccgggt caagaaggtc     300

gcgcgtgcga tcgaccatcc gctcgcgccg atcatcatcg gccaggacga ggcgccggtg     360

caggaagagg tcatcaccga tgacctcgac gtcaacaagt ggctgaccgc gatccgccac     420

acgccgcttg agaccgagct gacgatcggc tcaggcatct cgtgcgtcgt cggcccgtac     480

ttcgacggcg gcagccacat cggctacaac cgcatgaact tccgctgggg caacgtcggc 540

acgttccaga tctcgcccgg atcacacatg tggcaggtga tgaccgagca ctacaaggac 600

gacgagccga tcccgctgac gatgtgcttc ggcgtgcccc cgtcgtgcac cttcgttgcc 660

ggctcgggct tcgactacgc gatcctgccc aagggctgcg acgagatcgg catcgccggc 720

gcgatccagg gctcgccggt gcgcctcgtc aagtgccgca cgatcgacgc ctacacgctc 780

gccgacgccg agtacgtact cgaaggctat ctgcacccgc gcgacaagcg ctacgagacg 840

gccgagtcgg aagcggccga cgtccagggg cgctttcact tccacccgga gtgggcgggc 900

tacatgggca aagcgtacaa ggcgccgacg ttccacgtca ccgcgatcac gatgcgccgg 960

cgcgagagca agccgatcat cttcccgctc ggcgtgcaca cggccgacga cgccaacatc 1020

gacacctcgg tgcgcgagtc ggcgatcttc gcgctgtgcg agcggctgca gccgggcatc 1080

gtgcagaacg tgcatatccc gtactgcatg accgactggg gcggctgcat cattcaggtc 1140

aagaagcgca accagatcga ggaaggctgg cagcgcaatt tcctctccgc gatcctgtcg 1200

tgctcgcaag ggatgcgcct ggcgatcgcg gtgagcgaag acgtcgacat ctactcgatg 1260

gacgacatca tgtggtgtct gaccactcgc gtgaatccgc gcaccgacat cctcaacccg 1320

attcctggcg ggcgcggcca gaccttcatg ccggccgaac ggatgacctc gggcgacaag 1380

caatggaccg cctcgaacac ccagttcgaa ggcggcatgg ggatcgacgc gaccgtgccg 1440

ttcggctacg agagcgactt ccaccgcccg gtgtacggcg tcgatctggt caagccggaa 1500

gccttcttcg gcgagggcga catcaagaag atgcgctcgc gcatggaagg ctgggtgctg 1560

tccctggcac gcacgggacg ctga 1584

<210> 3
<211> 1188
<212> DNA
<213> Aromatoleum aromaticum

<400> 3
atgcttaacg cataccgggt cctggatctg agcaaccgca ccggctggct cgccggtcgg 60

ttgctcgccg atctcggggc cgacgtgatc aaggttgagg cgcggggggc caccctcgac 120

agcccggact ggcaggccta caacgtcaac aagcggctgt tgcgtctcga tctcgaaacg 180

cctgaaggcc ggttgggctt cgatcggctg ataggcggca tcgatatcct gatcgaatcg 240

gcaacgccgg gcgagcgcgc ccggtgcttg gatccgaccc ggctgcgcaa cctcaaccga 300

cgactgatcc acgtatcggt cacgccgttc ggctcgaccg gtccgcgctc cgactggctc 360

gcgtctgacc tcgagttaat ggccgccggc ggcgcgatgt cgcttgcggg cgagcccgac 420

gatgcgccca agcgcgtgac cgtgccacag tcgtactgct gggcgggggc gcaggcggcc 480

gtcggcgcgc tgaccgcgct cctccatcgc actgcctcag gtgaggggca gcacgtggac 540

gtatcggcgc aggccgcggt aatccttgcc ctgtcgcacg cgccggcctt ctgggacatg 600

gaaggcaccg ttccgacacg cgccggagcc tatgtcaccg ggcgctcgat caagggtgca 660

cgttatcgcg cgttctggcc ctgtgcggac ggctatctga acttcgtcct ctacggcggc 720

cccgcagggc gccgcacgaa tgcgcaactg atcgcgtgga tgcgcgagcg cggcgccgaa 780

ctcggggatc tcgcgcaaat cgactggaaa cgatttgacc cgaggcttgc gacgcaggaa 840

gaggtcgacg ggctggagcg gccgatcgcc gcattctttc tcggactcac aaagcgggaa 900

ttcctcgatc aggcgagtcg ccgcgagatg ctcggctacc cggtgtcgac gatgcatgac 960

attgccgacg acccgcaact ggcggcccgg gaattctggc acgacatgaa gacgccgaac 1020

ggggagtcgc agcggcattg cggcagtttt gcgattatcg accagctacg tgcgccgctt 1080

cgccatgagg cgggcgcgga aatcgatctc ggcgcattgc tggccgaact cggcctcgcc 1140

gctgcccgcc gagctgtcga tacggccgag gaggggcaat tcgtatga 1188

<210> 4
<211> 1221
<212> DNA
<213> Aromatoleum aromaticum

<400> 4
atgagcgcga tctctcaagc cctgtcgggc gtgaaagtgg cggaattcgg cgcgtatgcc 60

gcggggccac atatcggcaa gatgctcgcg accttcggcg caacggtcgt gcacgtagaa 120

tcccgtcagc gtcccgacgg gttccgcaac gagtacccgc cgtacaagga tggccgcccc 180

ggacccgatc ggagcggatg cttcgcgatc ttcaatgact ctaaatttga cgttacgctg 240

gacctcaaga ctcccgccgg agtcgaggtg gcgcagcgac tgatcacgtg ggccgacgtc 300

gtcatcgaga acatgcgccc cgacgtcatg tcgcgcctcg gccttggcta cgagacggca 360

cgcgagctga atccgggcgt cgtcatgatc tcgagctgca acatggggca aaccggtccg 420

cgcgcgcaga cgcccggctt cggctcgcag ctgtcggcgc ttgccggctt ttgcggcttg 480

accggtgatc ccaacggccc accgatgctg ctgtacggcc cgtatattga cttcatcgct 540

tcgacgctcg gcgccagcgc ggtcctcgcg gcactcgacc gtcagcgaag gataggccaa 600

ggcgcgtgga tcgatctggc gcagtacgaa agcgggctga tgtttgtcgc cggcgcgctg 660

ctcgattacc acaccaacgg ccgcgtggcg gaacgggcga acaacaccga ccccggcgca 720

gcaccacatg acgcttatca gtgcggcggc ggcacctggc tcgcgctttc gtgctggtcc 780

gacgaggagt tcgcgcgcct cgccgtggca ttgatgcgcc ccgatttgcc gggcaatcca 840

cgtttcgcga cgctggcggc gcgtaaggca aatattgcgg agctcgatgc aatcatttca 900

tcgtatctag tcacgcagga tgccgatgag gctgccgttc ggctccaggc agcgcaggtt 960

cacgcctatc cggtcaacac gattgctgac ctgttccggg atccgcaact cgcgtatcgc 1020

cggatctggc gccgtcgtct gcacggcgaa atcggggatg tcgcgtgtta tttctcggca 1080

ttcgatcttt cggtcacgcc cggcgatgtc atggctgcag cgccgtgcct cggccaggac 1140

aacgaactcg tattccggga atttctcgcc atgaccgacg tcgagtatgc ggaatatcag 1200

atcgaaggag cgttccagtg a 1221

<210> 5
<211> 981
<212> DNA
<213> Aromatoleum aromaticum

<400> 5
atgttacgaa acatcgtttg tggacttgcc attgcattct tcagcctcgg tgcagcggcg 60

gaatcctaca agatgaccct ttcgggggcg agccccagcg ggttgtggac gatgatcggc 120

gtaggtatag acggagccgt gaaagacgcc tttcctggat cgacgatcac ttatcagacg 180

tcgggtggag gactcgctaa tattgccctc ctcgatcagg gaaaagttga attgggaatc 240

gcgcacgacg ccgaactgca tattgccacc ctcggcgcca aacctttcag gaaacctatc 300

accaatttgc gtgcgatcgc gatgctgtat gactgggcgc caatgcagat ggtcatcacg 360

aaggcatttg cggaggagca cgggatccga acttttgacg atattgcggt gaagaaaccg 420

ccgatcagga ttgctttcaa caagcggggc aatatcaccg aatatgtcgc agtcgagatg 480

ttcagggcga tcggcgtgag cctggacgat atcaagaaat ggggcggcga tgtcatctat 540

gccgcatcgg acgaacaggg cgatctgatg aaggacaagc ggatcgatat gttcacaaac 600

ggggtattcg tgcgaacaag tttcatcgtc caggccggcg acgcggtcga cctcgttctg 660

ctcccagtgt cggacagcgt catcaaaaaa atctcccagg agctgagtgt cgccccgttt 720

gtcgtgaagg cgggaagcta ttcctggcaa cccaccgatg tcgcgaccct tgcactcagc 780

gcgatattgg tagtgaatga caagatggac gacaaggtcg cccatgatct ggcggaagcc 840

ttgcacaagc acattggaaa acttcaggga gcgcatggca gcctgaaagc cataactcca 900

gagttcctca cgagccagaa aactgtcccc taccacaagg gggccgaagc ttactaccgt 960

gaagctggac tactgaaata g 981

<210> 6
<211> 1971
<212> DNA
<213> Aromatoleum aromaticum

<400> 6
atggtctgca gacgctcctt gcattgggga gagccgagca tgtccagtat gagcctcgat 60

ttctttagca agtttttcac aaccggtgcg cgccgagatc ccgccagctg ggccggcaaa 120

cttctcaaac cctttgctgc gctggtcgcg ctgggcatcg tctgggtgac gaccatccag 180

attgtccaga tctatgcgat gacgatcgta ttcctcggtc tgatgctggc attggtcttt 240

ctgacaaccg gggcgaccgc agcctcgagc cgggagcggg ttccgctatg cgacctgcta 300

ttcgcagcgg cgagcatggg aatttccgcg tatttctggt ttcacaacga gcgcatcgtg 360

acccggatca cgctcctcga cgagctgact ttgtgggaca ttatttgcgg aagcgggctg 420

ctgctcatca ctctcgaagc gacacgacgc acggtcggcc cggggctgac gacgatcgtg 480

ctggtgtttc ttggctacaa tctgtggggc gaccgattgc ccggagtgct tggccacggt 540

tcgatcgact acctccactt tctcgatatc ctcgcgttca cgaccgacgg catctttggt 600

gcgccgatca gagtcgcttt gacctatgtc tttctgttcg gcctgttcgg aacctttctg 660

tcgcgtacgg gaggcggcga tttcttcttc gatgccgctt ccgccgtgag cggcaagagc 720

cccggcggtc ccgcgaaaat cgcggtggtt tcgtccggac tctacgggac catttccgga 780

agccccacgg ctgacgtggt cacgaccggg tcgatcacta ttccgatgat gaaaaagctc 840

ggctactcgg gcgcccttgc cggcggaatc gaagttgccg catccacggg cggcagcatc 900

ctgcctcctg tgatgggttc ggctgctttc atcatggccg agttcaccgg tatccgatat 960

acggatatcg cacttgcagg gatcctgccg gcattcctct actatttctg cgtctatctg 1020

caggtgcacc tcaactccct caaactggga atcgccggtc tcgacaaagt gccagccttc 1080

ggcgacacga tgcgacgagg agggatgttc ctggtcccgt tgatcgctct ggtggtggcg 1140

ctgctgattg gctattcccc caatttcgtc gccgcattcg gcaccctcgc agtgatcgcg 1200

gtggccatgt acaagcgcga gacccgtctc agtttcaagg ggttctatga gattctcgcg 1260

gaagcgactc tgcgagttgt gccggtcgtg gccgcctgtg ccgccgcggg gctggtcgtc 1320

ggcgggctgt cgatgaccgg actcggcgca aaggtgaccg aactcatctt cctccttgcc 1380

ggcacggatc tgtttttgtc gctggtggtc gccgcagtca tcacactggt gctgggcatg 1440

gggatgccga cccccagcgt gtacatcctc gccgcggtgc tggtcgcgcc ggcgctcacg 1500

aagctcggcg tttcgctgat cgctgcgcat ctgtttcttg tctattacgc tagcctgtcg 1560

gcgatgacac cgccgatcgc cgttgccgcg tttgccgctg ccccgatcgc gctcgccaac 1620

cctcttgccg tcggcctggc ggcggtgaga atggcgatga tcgcgttcgt ggttccgttt 1680

gccttcgtgt ataacaacgg cattctgctc tcgggagcgc cgctgcagat tgcatttgcc 1740

tgtctggcgg tgatccttgc cgtcgcctgc ctgtgtctgg cggccgaagg tttctggaaa 1800

cggccgctcg gcattgtgca gcgcttgtgt ttcctggtcg cgggactcgg cctgttgact 1860

ccctcgcctc aggtgcaggt agctggggga attgccgcag tattcggagc atggatggcg 1920

atacgtgtca gttcaggggt ggacgaccgt cacggaacat cgacgccttg a 1971

<210> 7
<211> 576
<212> DNA

<213>  Aromatoleum aromaticum

<400>  7
ttgattgctg aagagcaggt actcaaggat cctgtaaccc tgatggttac gcgggtgccg      60

aaacttgggc gggagacgga gtgggaagtc ctcatcggtg gaggcgttcg ggcggcgcgc     120

caattcccgg gaaacttggg tgccactatc ctcaaaccca ccgggaccgg ggaacgtgca     180

taccgaatca tcatgagttt cgatagcttc gcaagcctgc ggcgatggga ggactcggcg     240

caacggctgg aactggtgag gcaactcgaa tcagtggaga gcgcaccggt gctgatcgag     300

caggccgtcg gtttggaaac atggtttcag ttgccggcaa acgccggctc gccccagccg     360

atgattccac cgccccgcca caagatgatg atcgccagcg gactgggcgt gtatctgacg     420

atcacgcccc tgctgatgat cttgcgaccc gtgctcgaag ggctgccggt gtatatggct     480

acgatggtgc tgattccgat cgcggtggtc ctgctgacgt acgttgtgat gccggttatc     540

acgaaattgc tgcgccgctg gctgcgaggg gagtga                               576


<210>  8
<211>  8515
<212>  DNA
<213>  Artificial Sequence

<220>
<223>  pht cassette

<400>  8
   aagctttgac ctaaggaggt aaataatgaa aagactgata gtggggatca ctggggcgac      60
   gggggtggtg ttcggggttc ggttgctcga ggcgctgcag ggcaccgacg tggagacgca     120
   cctggtgctg agcaagtggg cgatccagac gatcgagcac gagacgtcct acacggccaa     180
   gcaggtgcgg gcgctcgccg cggtcgatca cgtcgaaggc aacatgggcg cgtcgatctc     240
   gtcgggctcg ttcgtgaccg aagggatggt gatcgcgccg tgctcgatga ggagcctcgc     300
   ggcgatcgcc cacggcacgg gcgaccacct ggtgcatcgg gcggccgacg tgatcctcaa     360
   ggagcggcgt cgcctggtgc tcgtgacgcg cgagatgccg ctctcggaca tccatctcga     420
   gaacatgctc aagctctcga agatgggcgt gacgatcatg ccgccgatgc ccgcgttcta     480
   taaccacccg cagacggtcg acgacatcgt ggatcacgtc gttgcccgga ttctcgatca     540
   attcggcctg ccggccgagt tcgcgcggcg ctgggagggg gagatgcgca accgcaaggt     600
   tgccaatctc cagccgcaga agtaatcgca cataccccag aaattcccga gtaaagcaac     660
   ctggaggcaa cacgatgaac gatctggcaa cgaaagaaat cgatgcggtg gccctgcggg     720
   ccggggtcaa ggacctgcgt gaagcgctcg agtggttccg cacccagggc tacctggtcg     780
   agaccgacaa ggaagtcaat cctgacctcg agatcaccgg tctgcagaag atcttcgacg     840
   gcagcctgcc gatgctcttc aacaacgtca aaggcatgcc ccatgcccgc gcgatcacga     900
   acctctttgg cgacatccgt gtcgtcgaag gcctcttcgg ctgggagaac tcgctcgacc 960
   gggtcaagaa ggtcgcgcgt gcgatcgacc atccgctcgc gccgatcatc atcggccagg 1020
   acgaggcgcc ggtgcaggaa gaggtcatca ccgatgacct cgacgtcaac aagtggctga 1080
   ccgcgatccg ccacacgccg cttgagaccg agctgacgat cggctcaggc atctcgtgcg 1140
   tcgtcggccc gtacttcgac ggcggcagcc acatcggcta caaccgcatg aacttccgct 1200
   ggggcaacgt cggcacgttc cagatctcgc ccggatcaca catgtggcag gtgatgaccg 1260
   agcactacaa ggacgacgag ccgatcccgc tgacgatgtg cttcggcgtg cccccgtcgt 1320
   gcaccttcgt tgccggctcg ggcttcgact acgcgatcct gcccaagggc tgcgacgaga 1380
   tcggcatcgc cggcgcgatc cagggctcgc cggtgcgcct cgtcaagtgc cgcacgatcg 1440
   acgcctacac gctcgccgac gccgagtacg tactcgaagg ctatctgcac ccgcgcgaca 1500
   agcgctacga gacggccgag tcggaagcgg ccgacgtcca ggggcgcttt cacttccacc 1560
   cggagtgggc gggctacatg ggcaaagcgt acaaggcgcc gacgttccac gtcaccgcga 1620
   tcacgatgcg ccggcgcgag agcaagccga tcatcttccc gctcggcgtg cacacggccg 1680
   acgacgccaa catcgacacc tcggtgcgcg agtcggcgat cttcgcgctg tgcgagcggc 1740
   tgcagccggg catcgtgcag aacgtgcata tcccgtactg catgaccgac tggggcggct 1800
   gcatcattca ggtcaagaag cgcaaccaga tcgaggaagg ctggcagcgc aatttcctct 1860
   ccgcgatcct gtcgtgctcg caaggatgcg gcctggcgat cgcggtgagc gaagacgtcg 1920
   acatctactc gatggacgac atcatgtggt gtctgaccac tcgcgtgaat ccgcgcaccg 1980
   acatcctcaa cccgattcct ggcggcgcgg gccagacctt catgccggcc gaacggatga 2040
   cctcgggcga caagcaatgg accgcctcga acacccagtt cgaaggcggc atggggatcg 2100
   acgcgaccgt gccgttcggc tacgagagcg acttccaccg cccggtgtac ggcgtcgatc 2160
   tggtcaagcc ggaagccttc ttcggcgagg gcgacatcaa gaagatgcgc tcgcgcatgg 2220
   aaggctgggt gctgtccctg gcacgcacgg gacgctgacc gctgctgcca ccgaaggagt 2280
   ccgcgatgct taacgcatac cgggtcctgg atctgagcaa ccgcaccggc tggctcgccg 2340
   gtcggttgct cgccgatctc ggggccgacg tgatcaaggt tgaggcgcgg ggggccaccc 2400
   tcgacagccc ggactggcag gcctacaacg tcaacaagcg gctgttgcgt ctcgatctcg 2460
   aaacgcctga aggccggttg ggcttcgatc ggctgatagg cggcatcgat atcctgatcg 2520
   aatcggcaac gccgggcgag cgcgcccggt gcttggatcc gacccggctg cgcaacctca 2580
   accgacgact gatccacgta tcggtcacgc cgttcggctc gaccggtccg cgctccgact 2640
   ggctcgcgtc tgacctcgag ttaatggccg ccggcggcgc gatgtcgctt gcgggcgagc 2700
   ccgacgatgc gcccaagcgc gtgaccgtgc cacagtcgta ctgctgggcg ggggcgcagg 2760
   cggccgtcgg cgcgctgacc gcgctcctcc atcgcactgc ctcaggtgag gggcagcacg 2820
   tggacgtatc ggcgcaggcc gcggtaatcc ttgccctgtc gcacgcgccg gccttctggg 2880
   acatggaagg caccgttccg acacgcgccg gagcctatgt caccgggcgc tcgatcaagg 2940
   gtgcacgtta tcgcgcgttc tggccctgtg cggacggcta tctgaacttc gtcctctacg 3000
   gcggccccgc agggcgccgc acgaatgcgc aactgatcgc gtggatgcgc gagcgcggcg 3060
   ccgaactcgg ggatctcgcg caaatcgact ggaaacgatt tgacccgagg cttgcgacgc 3120
   aggaagaggt cgacgggctg gagcggccga tcgccgcatt ctttctcgga ctcacaaagc 3180
   gggaattcct cgatcaggcg agtcgccgcg agatgctcgg ctacccggtg tcgacgatgc 3240
   atgacattgc cgacgacccg caactggcgg cccgggaatt ctggcacgac atgaagacgc 3300
   cgaacgggga gtcgcagcgg cattgcggca gttttgcgat tatcgaccag ctacgtgcgc 3360
   cgcttcgcca tgaggcgggc gcggaaatcg atctcggcgc attgctggcc gaactcggcc 3420
   tcgccgctgc ccgccgagct gtcgatacgg ccgaggaggg gcaattcgta tgagcgcgat 3480
   ctctcaagcc ctgtcgggcg tgaaagtggc ggaattcggc gcgtatgccg cggggccaca 3540
   tatcggcaag atgctcgcga ccttcggcgc aacggtcgtg cacgtagaat cccgtcagcg 3600
   tcccgacggg ttccgcaacg agtacccgcc gtacaaggat ggccgccccg gacccgatcg 3660
   gagcggatgc ttcgcgatct tcaatgactc taaatttgac gttacgctgg acctcaagac 3720
   tcccgccgga gtcgaggtgg cgcagcgact gatcacgtgg gccgacgtcg tcatcgagaa 3780
   catgcgcccc gacgtcatgt cgcgcctcgg ccttggctac gagacggcac gcgagctgaa 3840
   tccgggcgtc gtcatgatct cgagctgcaa catggggcaa accggtccgc gcgcgcagac 3900
   gcccggcttc ggctcgcagc tgtcggcgct tgccggcttt tgcggcttga ccggtgatcc 3960
   caacggccca ccgatgctgc tgtacgggcc gtatattgac ttcatcgctt cgacgctcgg 4020
   cgccagcgcg gtcctcgcgg cactcgaccg tcagcgaagg ataggccaag gcgcgtggat 4080
   cgatctggcg cagtacgaaa gcgggctgat gtttgtcgcc ggcgcgctgc tcgattacca 4140
   caccaacggc cgcgtggcgg aacgggcgaa caacaccgac cccggcgcag caccacatga 4200
   cgcttatcag tgcggcggcg gcacctggct cgcgctttcg tgctggtccg acgaggagtt 4260
   cgcgcgcctc gccgtggcat tgatgcgccc cgatttgccg ggcaatccac gtttcgcgac 4320
   gctggcggcg cgtaaggcaa atattgcgga gctcgatgca atcatttcat cgtatctagt 4380
   cacgcaggat gccgatgagg ctgccgttcg gctccaggca gcgcaggttc acgcctatcc 4440
   ggtcaacacg attgctgacc tgttccggga tccgcaactc gcgtatcgcc ggatctggcg 4500
   ccgtcgtctg cacggcgaaa tcggggatgt cgcgtgttat ttctcggcat tcgatctttc 4560
   ggtcacgccc ggcgatgtca tggctgcagc gccgtgcctc ggccaggaca acgaactcgt 4620
   attccgggaa tttctcgcca tgaccgacgt cgagtatgcg gaatatcaga tcgaaggagc 4680
   gttccagtga acaattgact agtatgttac gaaacatcgt ttgtggactt gccattgcat 4740
   tcttcagcct cggtgcagcg gcggaatcct acaagatgac cctttcgggg gcgagcccca 4800
   gcgggttgtg gacgatgatc ggcgtaggta tagacggagc cgtgaaagac gcctttcctg 4860
   gatcgacgat cacttatcag acgtcgggtg gaggactcgc taatattgcc ctcctcgatc 4920
   agggaaaagt tgaattggga atcgcgcacg acgccgaact gcatattgcc accctcggcg 4980
   ccaaaccttt caggaaacct atcaccaatt tgcgtgcgat cgcgatgctg tatgactggg 5040
   cgccaatgca gatggtcatc acgaaggcat ttgcggagga gcacgggatc cgaacttttg 5100
   acgatattgc ggtgaagaaa ccgccgatca ggattgcttt caacaagcgg ggcaatatca 5160
   ccgaatatgt cgcagtcgag atgttcaggg cgatcggcgt gagcctggac gatatcaaga 5220
   aatggggcgg cgatgtcatc tatgccgcat cggacgaaca gggcgatctg atgaaggaca 5280
   agcggatcga tatgttcaca aacggggtat tcgtgcgaac aagtttcatc gtccaggccg 5340
   gcgacgcggt cgacctcgtt ctgctcccag tgtcggacag cgtcatcaaa aaaatctccc 5400
   aggagctgag tgtcgccccg tttgtcgtga aggcgggaag ctattcctgg caacccaccg 5460
   atgtcgcgac ccttgcactc agcgcgatat tggtagtgaa tgacaagatg gacgacaagg 5520
   tcgcccatga tctggcggaa gccttgcaca agcacattgg aaaacttcag ggagcgcatg 5580
   gcagcctgaa agccataact ccagagttcc tcacgagcca gaaaactgtc ccctaccaca 5640
   agggggccga agcttactac cgtgaagctg gactactgaa atagatatga ccaatcagag 5700
   cggcggcgga taaattccac cgcgctccgg tccttgtcga gatgaattgc ggcaaggtca 5760
   ttgattgctc ttgggcggac ccgaataggt ctgtctgatc gtccctgcct gcagctccgg 5820
   tttatagatc tcgcgcgttc gttcatcttc gacgcgcgac cggaggtcca ttctgcacga 5880
   atggtctgca gacgctcctt gcattgggga gagccgagca tgtccagtat gagcctcgat 5940
   ttctttagca agtttttcac aaccggtgcg cgccgagatc ccgccagctg ggccggcaaa 6000
   cttctcaaac cctttgctgc gctggtcgcg ctgggcatcg tctgggtgac gaccatccag 6060
   attgtccaga tctatgcgat gacgatcgta ttcctcggtc tgatgctggc attggtcttt 6120
   ctgacaaccg gggcgaccgc agcctcgagc cgggagcggg ttccgctatg cgacctgcta 6180
   ttcgcagcgg cgagcatggg aatttccgcg tatttctggt ttcacaacga gcgcatcgtg 6240
   acccggatca cgctcctcga cgagctgact ttgtgggaca ttatttgcgg aagcgggctg 6300
   ctgctcatca ctctcgaagc gacacgacgc acggtcggcc cggggctgac gacgatcgtg 6360
   ctggtgtttc ttggctacaa tctgtggggc gaccgattgc ccggagtgct tggccacggt 6420
   tcgatcgact acctccactt tctcgatatc ctcgcgttca cgaccgacgg catctttggt 6480
   gcgccgatca gagtcgcttt gacctatgtc tttctgttcg gcctgttcgg aacctttctg 6540
   tcgcgtacgg gaggcggcga tttcttcttc gatgccgctt ccgccgtgag cggcaagagc 6600
   cccggcggtc ccgcgaaaat cgcggtggtt tcgtccggac tctacgggac catttccgga 6660
   agccccacgg ctgacgtggt cacgaccggg tcgatcacta ttccgatgat gaaaaagctc 6720
   ggctactcgg gcgcccttgc cggcggaatc gaagttgccg catccacggg cggcagcatc 6780
   ctgcctcctg tgatgggttc ggctgctttc atcatggccg agttcaccgg tatccgatat 6840
   acggatatcg cacttgcagg gatcctgccg gcattcctct actatttctg cgtctatctg 6900
   caggtgcacc tcaactccct caaactggga atcgccggtc tcgacaaagt gccagccttc 6960
   ggcgacacga tgcgacgagg agggatgttc ctggtcccgt tgatcgctct ggtggtggcg 7020
   ctgctgattg gctattcccc caatttcgtc gccgcattcg gcaccctcgc agtgatcgcg 7080
   gtggccatgt acaagcgcga gacccgtctc agtttcaagg ggttctatga gattctcgcg 7140
   gaagcgactc tgcgagttgt gccggtcgtg gccgcctgtg ccgccgcggg gctggtcgtc 7200
   ggcgggctgt cgatgaccgg actcggcgca aaggtgaccg aactcatctt cctccttgcc 7260
   ggcacggatc tgtttttgtc gctggtggtc gccgcagtca tcacactggt gctgggcatg 7320
   gggatgccga cccccagcgt gtacatcctc gccgcggtgc tggtcgcgcc ggcgctcacg 7380
   aagctcggcg tttcgctgat cgctgcgcat ctgtttcttg tctattacgc tagcctgtcg 7440
   gcgatgacac cgccgatcgc cgttgccgcg tttgccgctg ccccgatcgc gctcgccaac 7500
   cctcttgccg tcggcctggc ggcggtgaga atggcgatga tcgcgttcgt ggttccgttt 7560
   gccttcgtgt ataacaacgg cattctgctc tcgggagcgc cgctgcagat tgcatttgcc 7620
   tgtctggcgg tgatccttgc cgtcgcctgc ctgtgtctgg cggccgaagg tttctggaaa 7680
   cggccgctcg gcattgtgca gcgcttgtgt ttcctggtcg cgggactcgg cctgttgact 7740
   ccctcgcctc aggtgcaggt agctggggga attgccgcag tattcggagc atggatggcg 7800
   atacgtgtca gttcaggggt ggacgaccgt cacggaacat cgacgccttg acggacgaaa 7860
   ctcagcagca gaggagggag cgtgattgat tgctgaagag caggtactca aggatcctgt 7920
   aaccctgatg gttacgcggg tgccgaaact tgggcgggag acggagtggg aagtcctcat 7980
   cggtggaggc gttcggcggg cgcgccaatt cccgggaaac ttgggtgcca ctatcctcaa 8040
   acccaccggg accggggaac gtgcataccg aatcatcatg agtttcgata gcttcgcaag 8100
   cctgcggcga tgggaggact cggcgcaacg gctggaactg gtgaggcaac tcgaatcagt 8160
   ggagagcgca ccggtgctga tcgagcaggc cgtcggtttg gaaacatggt ttcagttgcc 8220
   ggcaaacgcc ggctcgcccc agccgatgat tccaccgccc cgccacaaga tgatgatcgc 8280
   cagcggactg ggcgtgtatc tgacgatcac gcccctgctg atgatcttgc gacccgtgct 8340
   cgaaggcctg ccggtgtata tggctacgat ggtgctgatt ccgatcgcgg tggtcctgct 8400
   gacgtacgtt gtgatgccgg ttatcacgaa attgctgcgc cgctggctgc gaggggagtg 8460
   aactagtcct gcaggttcta gagcggccgc caccgcggtg gagctccaat tcgcc 8515

<210> 9
<211> 50
<212> DNA
<213> Artificial Sequence

<220>
<223> Primer

<400> 9
ccaagctttg acctaaggag gtaaataatg aaaagactga tagtggggat 50

<210> 10
<211> 32
<212> DNA
<213> Artificial sequence

<220>
<223> Primer

<400> 10
ccactagttc actggaacgc tccttcgatc tg 32

<210> 11
<211> 73
<212> DNA
<213> Artificial Sequence

<220>
<223> Primer

<400> 11
gagtatgcgg aatatcagat cgaaggagcg ttccagtgaa caattgacta gtatgttacg 60

aaacatcgtt tgt 73

<210> 12
<211> 75
<212> DNA
<213> Artificial Sequence

<220>
<223> Primer

<400> 12
ggcgaattgg agctccaccg cggtggcggc cgctctagaa cctgcaggac tagttcactc 60

ccctcgcagc cagcg 75

<210> 13
<211> 18
<212> DNA
<213> Artificial Sequence

<220>
<223> Primer

<400>  13
agatcggcgt gatgttcg                                                    18


<210>  14
<211>  20
<212>  DNA
<213>  Artificial Sequence

<220>
<223>  Primer

<400>  14
accgaggcgt agaacttgag                                                  20


<210>  15
<211>  18
<212>  DNA
<213>  Artificial Sequence

<220>
<223>  Primer

<400>  15
ggcagcccaa ctacctga                                                    18


<210>  16
<211>  20
<212>  DNA
<213>  Artificial Sequence

<220>
<223>  Primer

<400>  16
tgcgcagata aatgtcatgg                                                  20


<210>  17
<211>  20
<212>  DNA
<213>  Artificial Sequence

<220>
<223>  Primer

<400>  17
cgcacagctt cgatgtctac                                                  20


<210>  18
<211>  19
<212>  DNA
<213>  Artificial Sequence

<220>
<223>  Primer

<400>  18
gcatcttgac gtggtcgaa                                                   19

<210>  19
<211>  19
<212>  DNA
<213>  Artificial Sequence

<220>
<223>  Primer

<400>  19
gcctcgacct ggatacctt                                                    19


<210>  20
<211>  21
<212>  DNA
<213>  Artificial Sequence

<220>
<223>  Primer

<400>  20
catctcgagt tcgaacagct c                                                 21


<210>  21
<211>  21
<212>  DNA
<213>  Artificial Sequence

<220>
<223>  Primer

<400>  21
aactcgacca actacgacgt g                                                 21


<210>  22
<211>  20
<212>  DNA
<213>  Artificial Sequence

<220>
<223>  Primer

<400>  22
gtcgtgcatc ttgacgtagc                                                   20

## Description

The invention relates to recombinant genetic cassettes and host cells comprising them which are useful as biotechnological tools for the aerobic and anaerobic biodegradation of phthalates and their bioconversion in value-added biodegradable polymers, such as polyhydroxyalkanoates (PHAs). Thus, the present invention belongs to the fields of microbiology and biotechnology, in particular to the industrially-relevant microbial platforms for the bioremediation of environments contaminated with phthalates, for plastic biowaste recycling and for the bioplastic biosynthesis.

### BACKGROUND ART

Phthalates are benzoic acids with one additional carboxylic group in either *ortho* (*o-*phthalate, PA), *meta* (isophthalate) or *para* (terephthalate) position. These compounds are produced from the bacterial degradation of polycyclic aromatic hydrocarbons such as phenanthrene, fluorene and fluoranthene found in fossil fuels. However, nowadays the main source of phthalates is the chemical industry. Phthalates are produced massively since they are essential constituents of plastics, either as part of the polymeric structure as in polyethylene terephthalate (PET), or as non-covalently dissolved additives within the polymeric structure to act as softeners or plasticizers. Most synthetic plasticizers are di-esters of PA and their global annual production is higher than 8 million tons (Junghare et al. 2016. Environ Microbiol, 18:3175-88). Therefore, high concentrations of PA can be found in soil around chemical factories. Since phthalate esters are not covalently bound to the plastic, they can easily diffuse out of the polymer itself, leading to contamination in nearly every environment. Thus, phthalate esters are one of the most frequently detected persistent organic contaminants in the environment, and they have been listed as major man-made priority pollutants due to their hepatotoxic, teratogenic, carcinogenic and endocrine disrupting (anti-androgenic) properties (Annamalai and Namasivayam. 2015. Environ Int. 2015;76:78-97). Hence, it becomes urgent to remove phthalates from the environment effectively and economically. On the other hand, and within the current strategies for plastic recycling as a sustainable plastic waste management, the treatment and valorization of the PA esters constitutes a major challenge.

Compared to abiotic degradation, microbial degradation of PA esters is a more efficient, cost-effective and environmentally friendly strategy. In most cases, degradation is initiated by non-specific esterases that release the free PA moiety and the side chain alcohols. Most often, side chain alcohols are easily utilized by bacteria, and PA accumulates (Liang et al. 2008. Appl Microbiol Biotechnol. 80:183-98). It is known that certain microorganisms can mineralize PA, as well as the other two phthalate isomers, under aerobic conditions. In all cases, the three phthalate isomers are converted to a dihydroxy benzoic acid, usually protocatechuate (3,4-dihydroxybenoate) but 2,3-dihydroxybenzoate has been also described, by the action of ring-hydroxylating dioxygenases and subsequent decarboxylation (Boll et al. 2020. Environ Microbiol Rep. 12:3-15; Kasai et al. 2019. Sci Rep. 9:1253).

In contrast to the well-studied aerobic degradation of PA, anaerobic degradation of PA was reported later in the literature and an initial activation to phthaloyl-CoA followed by decarboxylation to benzoyl-CoA was suggested as a putative degradation mechanism. However, only very recently studies based on differential proteomics and *in vitro* enzyme assays, allowed the identification and characterization of the genes (hereafter named as *pht*) and enzymes involved in the anaerobic peripheral pathway for the conversion of PA to benzoyl-CoA, which is further degraded through the anaerobic benzoyl-CoA central pathway (*bzd* genes). The pht peripheral pathway has been studied in closely related denitrifying beta-proteobacteria, such as *Azoarcus* sp. PA01 (Junghare et al., 2016 Environ Microbiol, 18: 3175-88), *Thauera chlorobenzoica* 3CB-1, *Aromatoleum aromaticum* EbN1 and *Aromatoleum evansii* KB740 (some aromatic compounds anaerobic degrading *Azoarcus* strains have recently been reclassified to the *Aromatoleum* genus) (Ebenau-Jehle et al., 2017 ISME J, 11: 224-36). Whereas in denitrifyers the PA is first activated to phthaloyl-CoA by a heterodimeric class III succinyl-CoA-dependent PA CoA transferase (SPT), in sulfate-reducing bacteria the activation is carried out by an ATP-dependent PA CoA ligase (PCL) (Geiger et al. 2019. Environ Microbiol. 21:3601-12). Both in denitrifying and sulfate-reducing bacteria, o-phthaloyl-CoA is subsequently decarboxylated to benzoyl-CoA by a two-component phthaloyl-CoA decarboxylase (PCD) belonging to the UbiD enzyme family of (de)carboxylases with its cognate UbiX prenyl-transferase that generates the prenylated flavin adenosine mononucleotide (prFMN) cofactor. Whereas the initial SPT enzyme is oxygen insensitive, the PDC enzyme from *T. chlorobenzoica* was oxygen sensitive (half-life in air of 13±3 min) in *in vitro* assays (Mergelsberg et al. 2017. Environ Microbiol. 19: 3734-44). However, PCD activity from *Azoarcus* sp. strain PA01 cells was not oxygen sensitive, although the strain was unable to grow aerobically on PA (Junghare et al. 2016. Environ Microbiol, 18:3175-88). Nevertheless, a detailed analysis of the genome sequence of strain PA01 revealed the absence of the *box* genes needed for the aerobic degradation of benzoyl-CoA, which could explain the lack of the aerobic growth of the strain in PA. *T. chlorobenzoica,* in contrast, contains a *box* cluster in its genome, but there are no data about its potential growth in PA under aerobic conditions. Thus, an extended role of the anaerobic pht pathway that might be also functional *in vivo* when denitrifying bacteria grow in PA in the presence of oxygen remains to be studied.

All PA-induced *pht* gene clusters described so far contain at least six genes (five genes in obligate anaerobes) encoding the catabolic enzymes SPT (*phtSab*) or PCL (*phtL*), and PCD (*phtDab*), as well as two additional genes (*phtTab*) proposed to encode a PA transporter (Boll et al., 2020 Environmental Microbiology Reports 12: 3-15). Uptake of PA inside bacterial cells appears to require an active transport system rather than passive diffusion across the inner membrane due to the high polar nature of this dicarboxylic acid. Among multicomponent solute-binding protein (SBP) dependent transport systems that rely on a periplasmic SBP protein which interacts with the substrate with high affinity, primary ATP-binding cassette (ABC) transporters for PA uptake have been characterized in *B. multivorans* ATCC 17616 (OphFGH) (Chang et al. 2009. J Bacteriol. 191:4671-3) and in *Rhodococcus jostii* RHA1 (PatABCD) (Hara et al. 2010. Appl Environ Microbiol. 76:1516-23). Secondary SBP-transport systems consist of a periplasmic SBP and usually two transmembrane proteins of different sizes, and they are not driven by direct ATP hydrolysis but by the proton motive force. The two major families of SBP-dependent secondary transporters are the tripartite ATP-independent periplasmic (TRAP) transporters and tripartite tricarboxylate transporters (TTT) (Rosa et al., 2018 Front Cell Infect Microbiol, 8: 33). Thus, in the aerobic degradation of PA, uptake of this dicarboxylic acid (as well as that of the other phthalate isomers) inside bacterial cells appears to require an active transport system rather than its passive diffusion across the inner membrane. However, there is no experimental demonstration that an active uptake is also essential for anaerobic PA degradation.

The degradation of PA through microorganisms has been approached in previous works, mostly by the identification and use of specific natural strains that degrade PA esters. The document CN103275909 discloses the gram-positive bacteria *Arthrobacter scleromae* C21 strain that degrades dimethyl PA. The document WO2006136173A2 relates to a process for anaerobic microbial degradation of phthalic esters using a composition of bacterial strains that degrade PAs, namely *Exiguobacterium gaetbuli* and *Bacillus fusiformis* strains. Despite such works, these are obviously limited by the growing conditions of the specific strains (such as aerobic vs. anaerobic) as well as by the lack of in-depth knowledge of the bacterial genes required for PA degrading, limiting a biosynthetic approach to PA degradation.

It becomes a great challenge to design biotechnological strategies able to remove or biodegrade phthalates from the environment in an effective, ecological and economic way. Moreover, within the current strategies for plastic recycling as a sustainable plastic waste management, the treatment and valorization of PAs towards the biosynthesis of bioplastics, for example PHB, is also desirable.

### DESCRIPTION OF THE INVENTION

The present invention provides an isolated, recombinant genetic cassette which comprises the genes involved in the *pht* pathway as well as the genes encoding an SBP-dependent secondary transporter. All of these genes are essential for microbial PA degradation, particularly for the conversion of PA to benzoyl-CoA. The invention also relates to recombinant host cells comprising this cassette and are thus useful for the aerobic or anaerobic biodegradation of phthalates, preferably PA, and for the bioconversion of PA in value-added biodegradable polymers, such as PHAs, preferably PHBs.

Thus, one aspect of the present invention relates to an isolated genetic cassette, from here onwards "the genetic cassette of the invention", comprising:
i. an UbiX-related FMN prenyltransferase gene (*phtDb*),
ii. an UbiD-related (de) carboxylase gene (*phtDa*),
iii. a subunit B of CoA-transferase of family III gene (*phtSb*),
iv. a subunit A of CoA-transferase of family III gene (*phtSa*),
v. a periplasmic binding protein gene (*phtTb*), and
vi. a membrane-component TRAP transporter gene (*phtTa*).

The terms "genetic cassette", "genetic construct" or "expression cassette" as used herein refer to any polynucleotide sequence, preferably a DNA unit, having a discrete and identifiable structure, a fragment or segment of DNA comprising a particular grouping of genetic elements, in the context of the present invention the encoding nucleotide sequences or genes specified in (i) to (vi) above. A genetic cassette can include one or more nucleic acid sequences. An example of a genetic cassette is one that contains one or more subunits of decarboxylase gene, one or more subunits of CoA-transferase gene, a periplasmic binding protein gene and a membrane subunit of a TRAP transporter gene.

A genetic cassette is therefore wherein a genetic material of interest can be introduced to be expressed as a protein or RNA in a host cell. The elements comprised within the genetic cassette are in reading frame or "operably linked", i.e. sequentially oriented therebetween in such a manner as to allow them to function in the intended manner, allowing the expression (i.e. the transcription and translation) of the encoding sequence(s) to take place in compatible conditions with the other elements or sequences present in the construct.

The genes comprised in the genetic cassette of the invention can be artificially obtained by conventional cloning and selection methods, or by sequencing. These genes may comprise other elements such as, for example, but not limited to, introns, noncoding sequences at 5' and/or 3' terminal ends, ribosome binding sites or stabilizing sequences. These polynucleotides may also include sequences that encode for additional amino acids that may be useful, for example, to increasing the stability of the peptide generated therefrom or enable simplified purification thereof.

The genes present in the genetic cassette of the invention allow the degradation of PA into benzoyl-CoA. This degradation pathway starts by the activation of PA to o-phthaloyl-CoA (Coenzyme A), which is carried out by the heterodimeric class III succinyl-CoA-dependent PA CoA transferase (SPT) enzyme in denitrifying bacteria. The SPT enzyme is formed by two subunits, A and B, which catalyze the aforementioned reaction. *o*-phthaloyl-CoA is subsequently decarboxylated to benzoyl-CoA by a phthaloyl-CoA decarboxylase belonging to the UbiD (Ubi of ubiquinone) enzyme family of (de)carboxylases with it cognate UbiX prenyl-transferase that generates the prenylated flavin adenosine mononucleotide cofactor. In order for the degradation of PA to initiate, PA needs to be uptake by the cell, requiring a multicomponent solute-binding protein (SBP) dependent transport system composed of a periplasmic binding protein which binds the PA with high affinity and a membrane component of a tripartite ATP-independent periplasmic (TRAP) transporter.

The genes indicated in (i) to (iv) above are *pht* catabolic genes and the genes indicated in (v) and (vi) are genes encoding a transporter that is essential for PA uptake in the cell.

In a preferred embodiment of the genetic cassette of the invention, the UbiX-related FMN prenyltransferase (*phtDb*) gene comprises a nucleotide sequence having at least 90% sequence identity with SEQ ID NO: 1, preferably 91, 92, 93, 94, 95, 96, 97, 98, 99 or 100% identity with the SEQ ID NO: 1. In a more preferred embodiment, the *phtDb* gene comprises the SEQ ID NO: 1. In an even more preferred embodiment, the *phtDb* gene consists of the SEQ ID NO: 1.

In another preferred embodiment of the genetic cassette of the invention, the UbiD-related (de) carboxylase (*phtDa*) gene comprises a nucleotide sequence having at least 90% sequence identity with SEQ ID NO: 2, preferably 91, 92, 93, 94, 95, 96, 97, 98, 99 or 100% identity with the SEQ ID NO: 2. In a more preferred embodiment, the *phtDa* gene comprises the SEQ ID NO: 2. In an even more preferred embodiment, the *phtDa* gene consists of the SEQ ID NO: 2.

In a further preferred embodiment of the genetic cassette of the invention, the subunit B of CoA-transferase of family III (*phtSb*) gene comprises a nucleotide sequence having at least 90% sequence identity with SEQ ID NO: 3, preferably 91, 92, 93, 94, 95, 96, 97, 98, 99 or 100% identity with the SEQ ID NO: 3. In a more preferred embodiment, the *phtSb* gene comprises the SEQ ID NO: 3. In an even more preferred embodiment, the *phtSb* gene consists of the SEQ ID NO: 3.

In yet another preferred embodiment of the genetic cassette of the invention, the subunit A of CoA-transferase of family III (*phtSa*) gene comprises a nucleotide sequence having at least 90% sequence identity with SEQ ID NO: 4, preferably 91, 92, 93, 94, 95, 96, 97, 98, 99 or 100% identity with the SEQ ID NO: 4. In a more preferred embodiment, the *phtSa* gene comprises the SEQ ID NO: 4. In an even more preferred embodiment, the *phtSa* gene consists of the SEQ ID NO: 4.

In a further preferred embodiment of the genetic cassette of the invention, the hypothetical protein, putative periplasmic binding protein gene (*phtTb*) comprises a nucleotide sequence having at least 90% sequence identity with SEQ ID NO: 5, preferably 91, 92, 93, 94, 95, 96, 97, 98, 99 or 100% identity with the SEQ ID NO: 5. In a more preferred embodiment, the *phtTb* gene comprises the SEQ ID NO: 5. In an even more preferred embodiment, the *phtTb* gene consists of the SEQ ID NO: 5.

In a more preferred embodiment of the genetic cassette of the invention, the membrane component of a TRAP transporter, 4TM/12TM fusion protein (*phtTa*), gene comprises a nucleotide sequence having at least 90% sequence identity with SEQ ID NO: 6, preferably 91, 92, 93, 94, 95, 96, 97, 98, 99 or 100% identity with the SEQ ID NO: 6. In a more preferred embodiment, the *phtTa* gene comprises the SEQ ID NO: 6. In an even more preferred embodiment, the *phtTa* gene consists of the SEQ ID NO: 6.

The term "identity", as used in this description, refers to the proportion of identical amino acids or nucleotides between two compared peptides/proteins or nucleotide sequences, respectively. The methods for comparing sequences are known in the state of the art, and include, but not limited to, the programs BLASTP or BLASTN, ClustalW and FASTA. We can consider that peptides, proteins or nucleotide sequences with percent identities of at least 90% will maintain the same properties as the sequence to which they refer.

The terms "nucleic acid" or "nucleotide" as used herein refer to nucleic acid molecules including DNA (gDNA, cDNA), RNA (eg. mRNA) and oligonucleotides (double or single stranded), and other such molecules as herein described. For the avoidance of doubt, the term "nucleic acid" includes non-naturally occurring modified forms, as well as naturally occurring forms.

In another preferred embodiment of the genetic cassette of the invention, it further comprises the *p2A95* gene comprising a nucleotide sequence having at least 90% sequence identity with SEQ ID NO: 7, preferably 91, 92, 93, 94, 95, 96, 97, 98, 99 or 100% identity with the SEQ ID NO: 7. In a more preferred embodiment, the *p2A95* gene comprises the SEQ ID NO: 7. In an even more preferred embodiment, the *p2A95* gene consists of the SEQ ID NO: 7.

In a more preferred embodiment of the genetic cassette of the invention, it comprises the SEQ ID NO: 8.

In another preferred embodiment of the genetic cassette of the invention, it further comprises the genes related to the benzoyl-CoA central pathway (*bzd* pathway). These genes include the cognate *bzdR* regulatory gene and the *bzdNOPQMSTUVWXYZA* catabolic genes.

In another preferred embodiment of the genetic cassette of the invention, it further comprises the genes related to the PHB pathway (*box* pathway), which comprise a β-ketothiolase (acetyl-CoA acetyltransferase) (encoded by *PhaA*), an acetoacetyl-CoA reductase (encoded by *PhaB1*), and a PHB synthase (encoded by *PhaC1*).

The genetic cassette of the invention can be introduced in a cloning or expression vector to enable its replication and expression in the interior of a host cell. Preferably, said vector is an appropriate vector for expressing the polypeptides encoded by the genes comprised in the genetic cassette of the invention.

Another aspect of the present invention relates to an expression vector, from here onwards "the vector of the invention", that comprises the genetic cassette of the invention.

The term "expression vector" as used herein refers to a nucleic acid molecule, preferably DNA, wherein the genetic cassette of the invention can be integrated without said vector losing its replication capacity. The expression vector is designed to direct the transformation or transduction of said genetic cassette in the interior of a target cell and its subsequent replication and expression. In addition to the genetic cassette of the invention, the vector comprises other genetic elements sequentially oriented (operably linked) in such a manner that the genetic cassette of the invention may be transcribed and translated in the cell. Said genetic elements may be, but not limited to, a promoter, a terminator, a leader sequence, a transcription initiation site, one or more replication origins, an untranslated regulatory region 3' and/or 5', a potentiator, a polyadenylation signal or any other control sequence.

The expression vector of the invention can be selected, but not limited to, from a plasmid, phage, cosmid, phagemid, autonomously replicating sequence (ARS), yeast artificial chromosome (YAC), bacterial artificial chromosome (BAC), human artificial chromosome (HAC) or viral vectors such as, but not limited to, lentivirus, baculovirus, adenovirus, retrovirus, adeno-associated viral vector (AAV) or any other type of DNA molecule capable of replicating in the interior of a prokaryotic or eukaryotic cell, preferably prokaryotic cell.

One type of preferred expression vector is a "plasmid", which refers to a circular double stranded DNA into which additional DNA segments may be cloned. In a preferred embodiment of the vector of the invention, the vector is a plasmid, here onwards the "plasmid of the invention".

The expression vector of the invention may be a self-replicating vector, whose replication is independent from the genome of the cell in which it is introduced, or may be a vector which becomes integrated in the genome of the target cell once transfected and replicates therewith. Thus, the vector may comprise sequences that facilitate its insertion in a specific location within the genome of the host cell. Certain vectors are capable of autonomous replication in a host cell into which they are introduced (e.g., bacterial vectors having a bacterial origin of replication and episomal mammalian vectors). Other vectors (e.g., non-episomal mammalian vectors) or parts thereof, are integrated into the genome of a host cell upon introduction into the host cell, and are thereby replicated along with the host genome. Moreover, certain vectors are capable of directing the expression of genes to which they are operatively linked. All these vectors are referred to herein as "recombinant expression vectors" or simply "expression vectors". In general, expression vectors of utility in recombinant DNA techniques are often in the form of plasmids. This disclosure is intended to include all forms of expression vectors, such as viral vectors (e.g., replication defective retroviruses, lentivirus, adenoviruses and adeno-associated viruses). The vector may also contain additional sequences, such as a "polylinker" or "multiple cloning site" for subcloning of additional nucleic acid sequences. A "vector backbone" or "plasmid backbone" refers to a piece of DNA containing at least a plasmid origin of replication and a selectable marker which allows for selection of hosts cells containing the plasmid or vector. When a particular plasmid or vector is modified to contain non-plasmid elements (e.g. insertion of Ad sequences and/or a eukaryotic gene of interest linked to a ribosomal promoter), the plasmid sequences are referred to as the plasmid backbone.

Preferably, the plasmid or vector of the present invention derives from the plasmid pIZ1016, in which the genes indicated in the present invention have been cloned.

In a preferred embodiment, the vector of the invention further comprises a reporter gene.

The term "reporter gene" as used herein refers to a nucleic acid encoding an identifying factor that is able to be identified based upon the reporter gene's effect, wherein the effect is used to track the inheritance of a nucleic acid of interest, to identify a cell, tissue, organ or organism that has inherited the nucleic acid of interest, and/or to measure gene expression induction or transcription. Selectable marker genes may also be considered reporter genes. Thus, the "reporter gene" to which the present invention relates may be any gene whose expression product can be identified, detected and/or quantified due to the fact that it provides an identifiable change in the cell that expresses it. Thus, the presence and expression of the marker gene in the genetic cassette of the invention will make it possible to easily identify the cells that comprise said cassette.

Examples of reporter genes known and used in the art can be elected from known databases such as FPbase (https://www.fpbase.org) or similar. Said reporter gene may be, for example, but not limited to, a gene that confers resistance to metals or to medicaments, preferably to antibiotics, such as for example genes conferring resistance to neomycin, gentamicin, puromycin, hygromycin, DHFR, GPT, zeocin, histidinol, streptomycin, ampicillin or similar. Inducible or non-inducible reporter genes, such as GFP, EGFP, mCherry, Cyan fluorescent protein (CFP), Yellow fluorescent protein (YFP), luciferase (Luc), IRV3, or any other fluorescent or chemo or bioluminiscent protein, beta-galactosidase (LacZ), β-glucuronidase (Gus), chloramphenicol acetyltransferase (CAT), horseradish peroxidase (HRP) or similar. Encoding marker genes can also be used for epitopes or immunogenic molecules, such as for example, but not limited to, FLAG, HA, His, Myc, V5, Xpress, Thrombin, DAD, S Tag, C Protein. The selection of the marker gene is not relevant provided that it can be simultaneously expressed with the genes indicated in elements (i) and (vi) of the genetic cassette of the invention.

In a more preferred embodiment of the vector of the invention, the vector further comprises control sequences operationally linked to the genetic cassette of the invention.

The term "control sequences" as used herein refers to nucleic acid sequences which allow the expression of the genetic cassette of the invention and to control the transcription. Such control sequences include, but are not limited to, a polyadenylation signal, a promoter (e.g., natural or synthetic promotor) or an enhancer to effect transcription, an optional operator sequence to control transcription, a locus control region or a silencer to allow a tissue-specific transcription, a sequence encoding suitable ribosome-binding sites on the mRNA, a sequence capable to stabilize the mRNA and sequences that control termination of transcription and translation. These control sequences can be modified, e.g., by deletion, addition, insertion or substitution of one or more nucleic acids, whereas saving their control function. Other suitable control sequences are well known in the art.

The term "promoter" as used herein refers to a polynucleotide, generally located "upstream" of the starting point of the transcription, required for transcription at significant levels of a second polynucleotide to which it is operably combined. It is capable of initiating the transcription of a nucleotide sequence in a cell. This term includes, for example, constitutive or inducible/repressible promoters. Preferably, the promoter to which the invention relates is constitutive. The origin of the selected promoter will depend on the cell in which the genetic cassette of the invention is intended to be expressed. Thus, promoters of eukaryotic, prokaryotic or viral origin may be used. Examples of prokaryotic promoters include, but not limited to, *E. coli* trp, recA, lacZ, lacI, tet, gal, trc, or tac gene promoters, or the *B*. *subtilis* α-amylase gene promoter. Examples of viral promoters are, but not limited to, the cytomegalovirus promoter, the SV40 promoter, of the Rous sarcoma virus LTR, of the murine leukemia virus LTR or of the herpes simplex virus thymidine kinase. Examples of eukaryotic promoters are, but not limited to, the human beta actin promoter, TRE, UAS, Ac5, of polyhedrin, CaMKIIIa, GAL1, 10, TEF1, GDS, ADH1, CaMV35S, H1, U6, B29, of CD14, of CD43, of CD45, of CD68, of desmin, of elastase-1, of endoglin, of fibronectin, of GFAP, of Flt-1, EF1alfa, PGK, CAG or UBC. Promoters for use in bacterial systems also will contain a Shine-Dalgarno sequence operably linked to the DNA encoding the desired polypeptides. The most preferred promoters are selected from the list consisting of: lacl-Plac, lacl-Ptac, cl-Pr xyIS-Pm, alkS-Palk, araC-Pbad, chnR-PchnB, cprK1-PDB3 and PEM7.

The terms "operationally linked" or "operational combined" as used herein refer to that separate nucleic acid sequences are functionally associated such that an event at one can precipitate a response from the other. Two or more operably linked nucleic acid sequences can, in combination, comprise an independent genetic element, such as an expression cassette. Operational linked means that the nucleic acid sequence is linked to the promoter in a manner which allows expression (e. g., transcription and/or translation) of the nucleic acid sequence. Transcription means the process whereby information contained in a nucleic acid sequence of DNA is transcribed to complementary RNA sequence.

Another aspect of the invention refers to a recombinant host cell, hereinafter "the host cell of the invention", comprising, heterologously (i.e. artificially inserted), the genetic cassette or the expression vector of the invention.

The recombinant host cell of the invention has been transduced, transfected, transformed or similar with the genetic cassette or expression vector of the invention using any genetic engineering technique from those known in the art for such purpose. Said techniques may be, for example, but not limited to, injection or microinjection, *ex vivo* transformation, electroporation, precipitation with calcium phosphate, DEAE-dextrane followed by polyethylene glycol, sonication, biolistics, retroviral infection, liposome-mediated transfection (lipofection) or via receptor, or other techniques widely known in the state of the art.

The terms "transfection", "transduction" and "transformation" are used herein interchangeably to define the methods whereby the genetic material of a cell has been altered, preferably increased, through the introduction of the genetic cassette or expression vector of the invention using genetic engineering techniques.

In a preferred embodiment, the host cell of the invention further comprises endogenously (i.e. naturally), the genes related to the *bzd* pathway or the genes related to the box pathway.

Examples of cells comprising endogenously the genes related to the *bzd* pathway are *Aromatoleum* or *Azoarcus, Thauera, Sulfuritalea, Herminiimonas,, Magnetospirillum, Geobacter, Syntrophus, Rhodopseudomonas, Desulfococcus, Desulfitobacterium, Pelotomaculum, Desulfotomaculum, Sedimenticola, Dechloromarinus and Ferroglobus.*

Examples of cells comprising endogenously the genes related to the box pathway are *Aromatoleum* or *Azoarcus, Thauera, Cupriavidus, Burkholderia, Ramlibacter, Comamonas, Sulfuritalea, Ralstonia, Delftia and Zoogloea.*

In another preferred embodiment, the host cell of the invention is a bacterial cell. More preferably, the bacterial cell belongs to the *Cupriavidus* or *Azoarcus*/*Aromatoleum* genus, even more preferably the cell belongs to the *Azoarcus* sp. CIB strain or the *Cupriavidus necator* H16 strain.

The terms *Aromatoleum* and *Azoarcus* are synonymous. The terms *Cupriavidus necator* and *Ralstonia eutropha* are synonymous.

Another aspect of the invention refers to a method for producing the recombinant host cell of the invention which comprises the insertion of the genetic cassette or the expression vector of the invention into a suitable host cell.

Another aspect of the invention refers to the use of the genetic cassette or the expression vector of the invention for generating a cell, preferably the host cell of the invention, capable of degrading phthalates, preferably *o*-phthalate (PA), more preferably capable of the bioconversion of PA into polyhydroxyalkanoates (PHAs), preferably polyhydroxybutyrate (PHB).

The host cell of the invention, comprising the genetic cassette or the expression vector of the invention, is capable of using PA as a carbon source and converting it into benzoyl-CoA, which may be further degraded to PHB if the anaerobic *bzd* pathway or the aerobic *box* pathway is further present (naturally or artificially) in the cell.

Thus, another aspect of the invention refers to the use of the genetic cassette, the expression vector or the recombinant host cell of the invention for the (bio)degradation of phthalates, preferably o-phthalate (PA). This degradation may be carried out under aerobic or anaerobic conditions. Preferably, this degradation is carried out aerobically.

The use of the genetic cassette, the expression vector or the recombinant host cell of the invention for the (bio)degradation of phthalates, preferably *o*-phthalate (PA) via aerobic degradation has the advantage, compared to classical aerobic pathways, of not depending on ring-hydroxylating dioxygenases which require high oxygen tensions that may quickly become limiting in many natural ecosystems or when bacteria grow on surfaces forming biofilms.

Another aspect of the invention refers to the use of the genetic cassette, the expression vector or the recombinant host cell of the invention for the bioconversion of PA into polyhydroxyalkanoates (PHAs), preferably polyhydroxybutyrate (PHB). This use refers to the genetic cassette or expression vector of the invention when they further comprise the genes related to the *bzd* pathway or the genes related to the *box* pathway; or to the host cell of the invention when it comprises the genetic cassette of the invention and further, endogenously, the genes related to the *bzd* pathway or the genes related to the *box* pathway. When the genes related to the *bzd* pathway are present, this bioconversion is carried out anaerobically. When the genes related to the *box* pathway are present, this bioconversion is carried out aerobically.

Unless otherwise defined, all technical and scientific terms used herein have the same meaning as commonly understood by one of ordinary skilled in the art to which this invention belongs. Methods and materials similar or equivalent to those described herein can be used in the practice of the present invention. Throughout the description and claims the word "comprise" and its variations are not intended to exclude other technical features, additives, components, or steps. Additional objects, advantages and features of the invention will become apparent to those skilled in the art upon examination of the description or may be learned by practice of the invention. The following examples, drawings and sequence listing are provided by way of illustration and are not intended to be limiting of the present invention.

### DESCRIPTION OF THE DRAWINGS

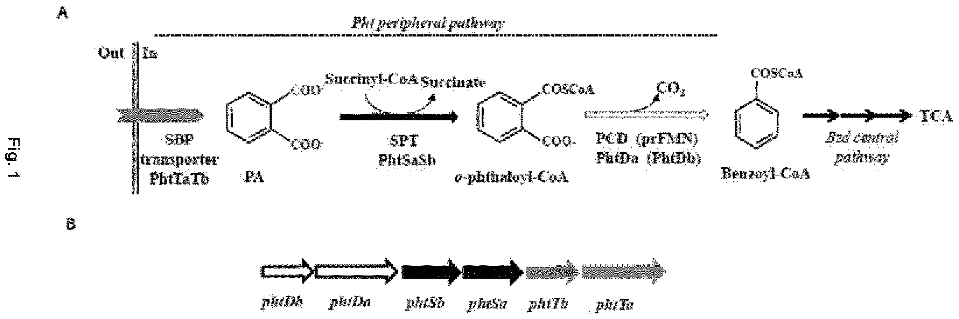

**Fig. 1****. Scheme of the biochemistry and associated genetic determinants of the peripheral *pht* pathway for anaerobic PA degradation in denitrifying bacteria. A)** Uptake of PA inside the bacterial cell by a putative solute-binding protein (SBP) transporter (PhtTaTb) is indicated with a grey arrow. Activation of PA to o-phthaloyl-CoA by the SPT CoA transferase (PhtSaSb) is shown with a black arrow. Decarboxylation of o-phthaloyl-CoA to benzoyl-CoA by a PCD decarboxylase (PhtDa and its associated PhtDb FMN prenyltransferase) is shown with a white arrow. Benzoyl-CoA is finally de-aromatized and funneled into the tricarboxylic acid cycle (TCA) via the anaerobic bzd central pathway. **B)** General organization of the PA-induced *pht* gene cluster.

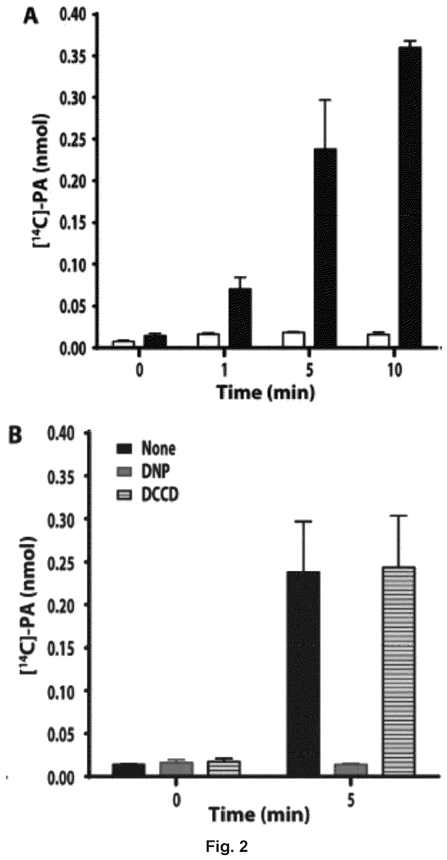

**Fig. 2****. Uptake of PA by *A. evansii* cells grown under nitrate-reducing conditions. A)** The [¹⁴C]-PA uptake assays were performed with cells grown in benzoate (white bars) or PA (black bars). **B)** Effect of energetic inhibitors on [¹⁴C]-PA uptake in *A. evansii* cells grown anaerobically in PA. Cells were grown and then exposed or not (None) to 1 mM DNP (proton-motive force blocking agent 2,4-dinitrophenol) or 1 mM DCCD (ATP synthase inhibitor dicyclohexylcarbodiimide) before performing the [¹⁴C]-PA uptake assays. Graphed values are the average of three independent experiments (error bars indicate standard deviations).

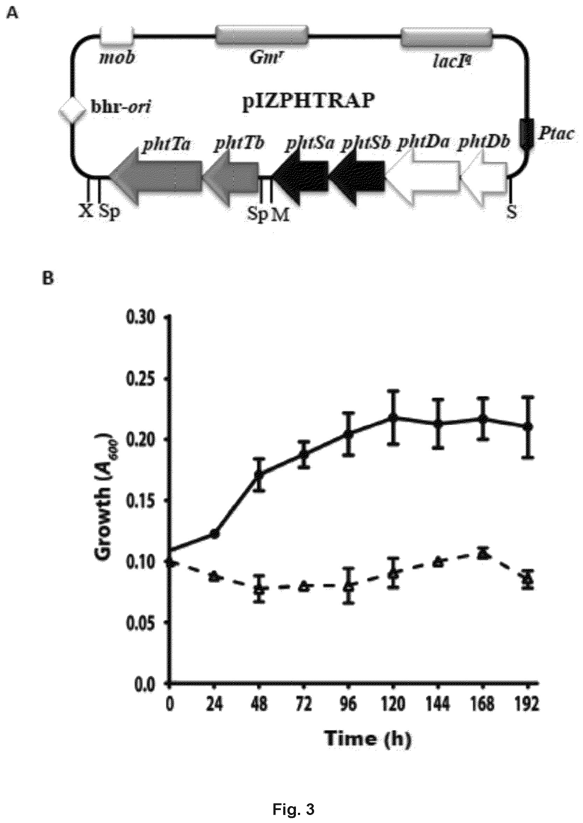

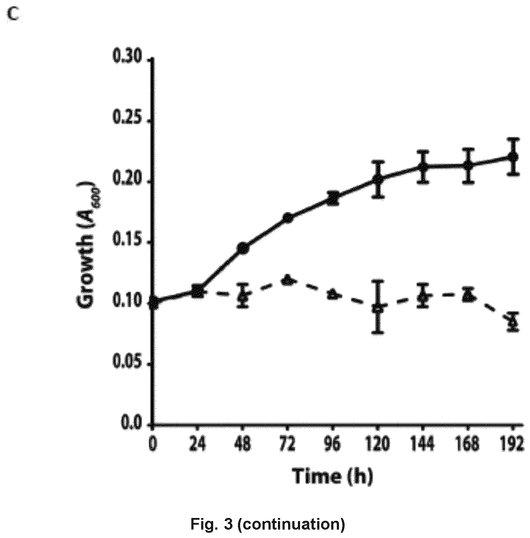

**Fig. 3****. Expression of the *pht* cassette in *Azoarcus* sp. CIB cells. A)** Scheme of the *pht* cassette cloned into the broad-host range plZPHTRAP plasmid. The *pht* genes are indicated by white, black and grey thick arrows. Some restriction sites are indicated: M, MfeI, S, SgrDI, Sp, SpeI, X, XbaI. The gentamicin resistance gene (*Gm^{r}*), mobilization (*mob*), broad-host range origin of replication (bhr ori), and the regulatory couple *lac*I^{q} repressor gene *(lacI^{q}*) and *Ptac* promoter are shown. **B)** Growth curves of *Azoarcus* sp. CIB (pIZPHTRAP) (filled circles) and *Azoarcus* sp. CIB (pIZPHTRAPΔ*phtDa*) (open triangles). Cells were grown anaerobically in MC minimal medium containing 3 mM PA and 10 mM nitrate as sole donor and electron acceptors, respectively. Gentamicin (7.5 µg ml⁻¹) was added to assure plasmid maintenance. Bacterial growth was monitored by measuring *A*₆₀₀. **C)** Growth curves of *Azoarcus* sp. CIB (pIZPHTRAP) (filled circles) and *Azoarcus* sp. CIB pIZPHTRAPΔ*phtDa* (open triangles) cultivated aerobically in MC minimal medium containing 3 mM PA. Values are the mean of three different experiments. Error bars indicate standard deviations.

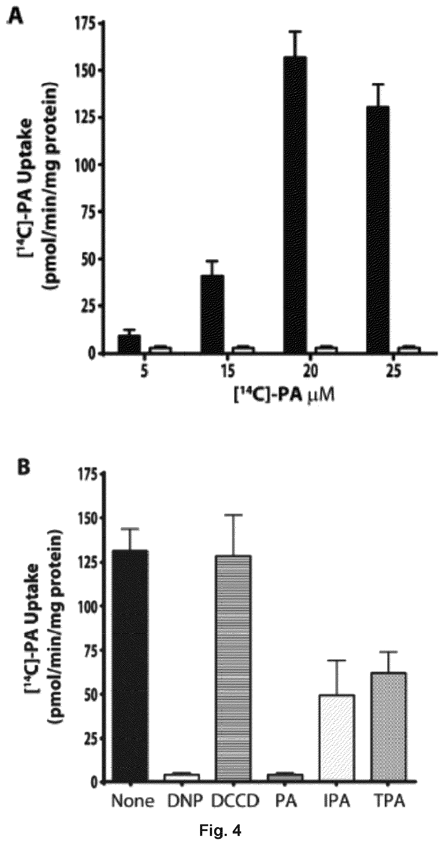

**Fig. 4****. Uptake of PA by the PhtTa-PhtTb TAXI-TRAP transporter. A)** [¹⁴C]-PA uptake assays were performed with *Azoarcus* sp. CIB (pIZPHTRAP) cells expressing the PhtTa-PhtTb transporter (black bars) and with *Azoarcus* sp. CIB (pIZPHT) control cells that do not express the PhtTa-PhtTb transporter (white bars), by using increasing concentrations of [¹⁴C]-PA from 5 to 25 µM). **B)** The uptake rates of [¹⁴C]-PA were examined by pre-incubating cells for 10 min with either 1 mM DNP or 1 mM DCCD previous to the transport assay. PA uptake rates were also examined by adding 250 µM non-labelled PA, isophthalate (IPA), or terephthalate (TPA) as competitors to the transport assay containing 25 µM [¹⁴C]-PA. Values are the mean of three different experiments. Error bars indicate standard deviations.

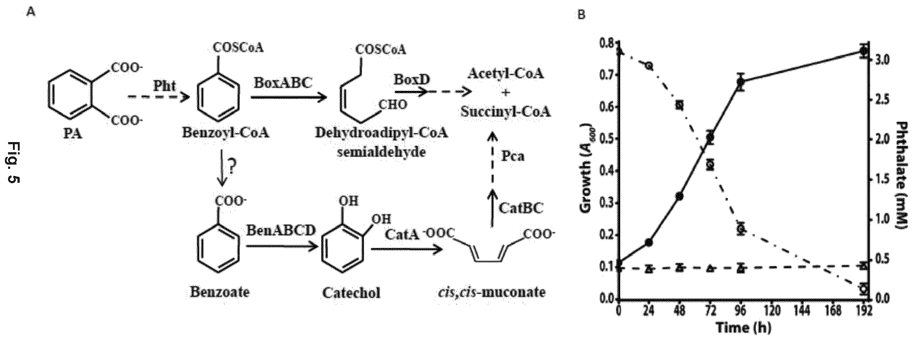

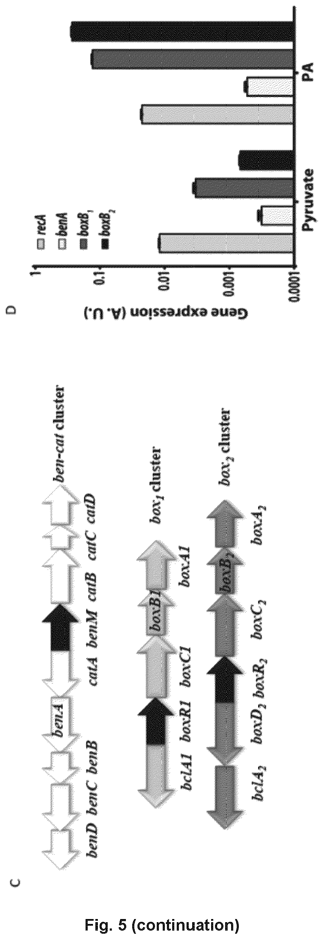

**Fig. 5****. Expressing the *pht* cassette in *C. necator* H16 cells. A)** Scheme of the putative PA degradation pathways in *C. necator* H16 cells expressing the Pht enzymes that converts PA to benzoyl-CoA. The aerobic hybrid pathway (*box* pathway) and the classical aerobic pathway (*ben-cat* pathway) are indicated. Main enzymes and intermediates are shown. Discontinuous arrows indicate that more than one enzymatic step is involved. **B)** Growth curves of *C. necator* H16 cells (dots and dashes line) and *C. necator* H16 (pIZPHTRAP) (black solid line) in MC minimal medium containing 3 mM PA. Bacterial growth was monitored by measuring *A*₆₀₀. Consumption of PA is also indicated (dashes line). Values are the mean of three different experiments. Error bars indicate standard deviations. **C)** Organization of the *ben-cat* cluster and the two *box* clusters in the genome of strain H16. The *benA, boxB₁* and *boxB₂* genes used as probes for monitoring the expression of the *ben-cat, box1* and *box2* clusters, are highlighted. Black arrows show the regulatory genes. **D)** Expression of the housekeeping *recA* gene, and that of *benA, boxB₁* and *boxB₂* genes when *C. necator* H16 (pIZPHTRAP) cells are grown in 18 mM pyruvate or 3 mM PA. Total RNA was isolated and the expression of the genes was measured by real-time RT-PCR. The relative expression of the genes is shown in arbitrary units (AU). Each value is the average from three separate experiments; error bars indicate standard deviations.

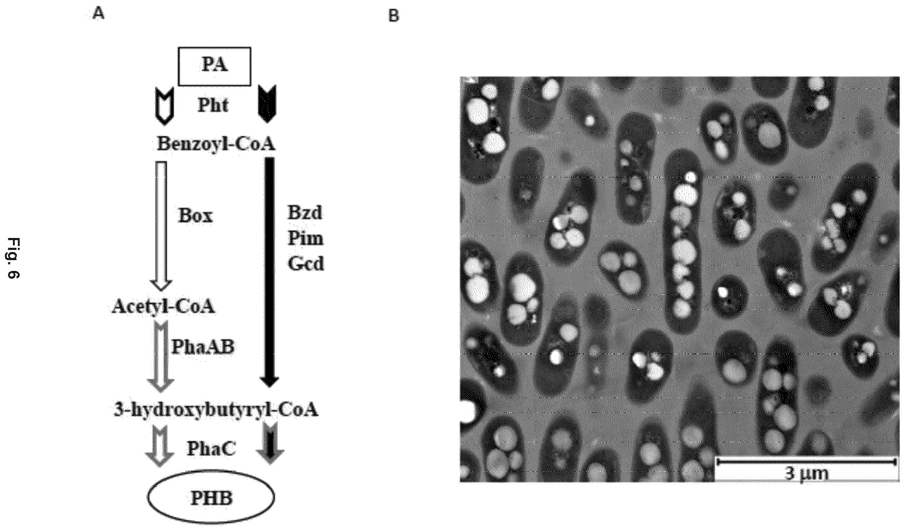

**Fig. 6****. Funneling PA towards the synthesis of polyhydroxybutyrate (PHB) in biocatalysts expressing the *pht* cassette. A)** Scheme of the metabolic pathways involved in the aerobic (white arrows) and anaerobic (black arrows) conversion of PA to PHB. Abbreviations. Pht, Pht peripheral pathway; Box, hybrid aerobic pathway; Bzd, anaerobic benzoyl-CoA central degradation pathway; Pim-Gcd, benzoyl-CoA lower pathway; PhaAB, enzymes involved in the conversion of acetyl-CoA into the monomeric precursor of PHB, i.e., 3-hydroxybutyryl-CoA. PhaC, PHB synthase. **B)** Representative transmission electron microscopy (TEM) view of *Azoarcus sp.* CIB (pIZPHTRAP) cells grown anaerobically on PA. PHB granules can be observed as white spheres inside the cells.

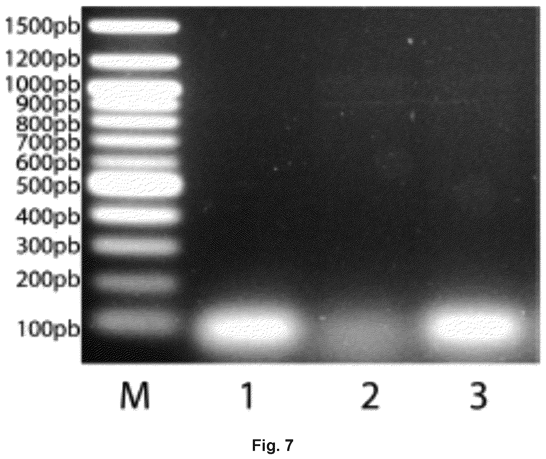

**Fig. 7****. Expression of the *boxB* gene in aerobically-grown *Azoarcus* sp. CIB (pIZPHTRAP) cells.** Cells were grown under oxic conditions in MC minimal medium with 3 mM benzoate (lane 1), 0.2% pyruvate (lane 2) or 3 mM PA (lane 3) as sole carbon source. Total RNA was isolated from cells grown until the end of the exponential phase, and the expression of the *boxB* gene was monitored by RT-PCR. Agarose gel electrophoresis of RT-PCR products is shown. Lane M, molecular size markers (Quick-Load™ 100bp DNA Ladder from New England BioLabs).

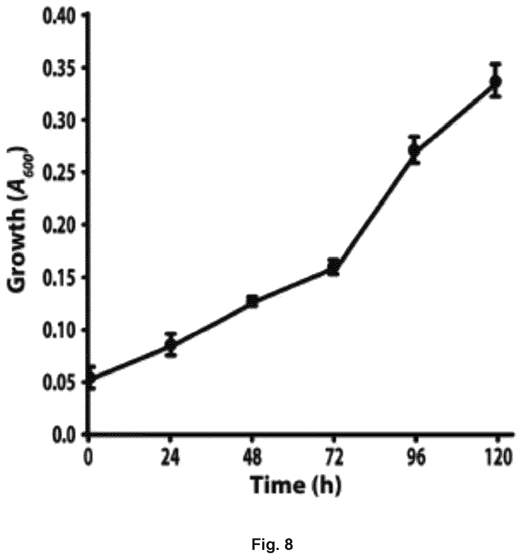

**Fig. 8****. Growth curve of *A. evansii* cultivated aerobically in MC minimal medium containing 3 mM PA as sole carbon source.** Bacterial growth was monitored by measuring *A*₆₀₀. Values are the mean of three different experiments. Error bars indicate standard deviations.

### EXAMPLES

### Example 1 - Materials and Methods

### Strains, plasmid and growth conditions

The *Escherichia coli, Pseudomonas, Aromatoleum* and *Cupriavidus* strains as well as the plasmids used in this work are listed in Table 1.

**Table 1**

| Strain or plasmid | Relevant genotype and main characteristics | Reference or source |
|---|---|---|
| *E. coli* strains | | |
| DH10B | *F', mcrA, Δ*(*mrr hsdRMS-mcrBC), Φ80lacZΔM15, ΔlacX74, deoR, recA1, araD139, Δ(ara-leu)7697, galU, galK, rpsL (SmR), endA1, nupG* | Life Technologies |
| S17*-1λpir* | *Tpr Smr recA thi hsdRM*+ *RP42::Tc::Mu::Km Tn7 Apir phage lysogen* | (de Lorenzo and Timmis 1994, Methods Enzymol, 235, 386-405) |

| *Azoarcus*/*Aromatoleum* strains | | |
|---|---|---|
| *Azoarcus sp.* CIB | *Wild-type CIB strain, PA-* | Lopez Barragan et al. 2004, Journal of Bacteriology 186(17):5762-74 |
| *Aromatoleum evansii* | *Wild-type strain, PA+* | Anders HJ *et al.,* Int J Syst Bacteriol. 1995;45:327-33 |

| *Cupriavidus* strains | | |
|---|---|---|
| *Cupriavidus necator* H16 | *Wild-type strain, PA-* | Pohlmann et al. 2006, Nat Biotechnol, 24: 1257-62 |
| *Cupriavidus pinatubonensis* | *Wild-type strain, PA-* | Don, HR et al., J Bacteriol. 1981; 145:681-6. |

| *Pseudomonas* strains | | |
|---|---|---|
| *Pseudomonas putida* KT2440 | *Wild-type strain, PA-* | Franklin et al., Proc Natl Acad Sci U S A. 1981;78:7458-62 |

| Plasmids | | |
|---|---|---|
| plZ1016 | *Gmr, pBBR1MCS-5 broad-host range cloning vector* | Moreno-Ruiz et al. 2003, J Bacteriol, 185: 2026-30 |
| plZPHT | *Gmr, plZ1016 derivative harbouring the catabolic pht genes for the peripheral PA degradation pathway from Aromatoleum aromaticum EbN1 strain.* | This work |
| plZPHTRAP | *Gmr, pIZ1016 derivative harbouring the pht cassette for the peripheral PA degradation pathway* | This work |
| plZPHTRAPΔphtDa | *Gmr, pIZPHTRAP derivative lacking the phtDa gene* | This work |

*E. coli* cells were grown at 37 °C in Luria-Bertani (LB) medium (Miller 1972)). *Azoarcus* strains were grown at 30 °C anaerobically in MC medium using the indicated carbon source(s) and 10 mM nitrate as the terminal electron acceptor, as described previously (Lopez Barragan et al. 2004, J Bacteriol, 186: 5762-74). *Azoarcus* and *Cupriavidus* strains were grown at 30°C aerobically in NB (Difco, 234000) or in MC (without FeS and nitrate). The strains *Azoarcus* and *Cupriavidus* carrying the plasmids (pIZPHT, pIZPHTRAP and pIZPHTRAPΔDβ) were grown overnight at 30°C in NB with gentamycin (7.5 µg/ml) and 1 mM of Isopropyl β-D-1-thiogalactopyranoside (IPTG) to induce the heterologous gene expression until 1.8≈2.0 optical density (OD); were centrifugated, 20 min at 4°C, harvest and resuspended in sodium salt before inoculate at OD 0.1 in MC with 3mM of o-phthalate as carbon source, without gentamycin. In order to rule out possible contaminations due to the absence of antibiotic, bacterial growth as *Azoarcus* or *Cupriavidus* strains were checked by 16S rRNA gene sequencing.

### Molecular Biology Techniques

Recombinant DNA techniques were carried out by published methods (Sambrook 2001, Third edition. Cold Spring Harbor, N.Y.: Cold Spring Harbor Laboratory Press). Plasmid DNA was prepared with a High Pure plasmid isolation kit (Roche Applied Science). DNA fragments were purified with Gene-Clean Turbo (Q-BIOgene). Oligonucleotides were synthesized on an Oligo-1000 M nucleotide synthesizer (Beckman Instruments, Inc.). Gibson Assembly was performed following the published methods (New England Biolabs). All cloned inserts and DNA fragments were confirmed by DNA sequencing through an ABI Prism 377 automated DNA sequencer (Applied Biosystems Inc.). Transformation of *E. coli* cells was carried out by using the RbCI method or by electroporation (Gene Pulser; Bio-Rad) (Sambrook 2001, Third edition. Cold Spring Harbor, N.Y.: Cold Spring Harbor Laboratory Press). Plasmids were transferred from *E. coli* S17*-*1*λpir* (donor strain) into *Azoarcus sp.* recipient strains by biparental filter mating as described previously (Lopez Barragan et al. 2004, J Bacteriol, 186: 5762-74). The protein concentration in cell extracts was determined by the method of Bradford by using bovine serum albumin as the standard.

### Construction of pht cassette and derivatives

The oligonucleotides used in this study are listed in Table 2.

The plZPHT plasmid was constructed by amplifying the region 66.351-71015 bp of plasmid 2 from *A. aromaticum* EbN1 with the EbN1 113 FW/ EbN1 102 RV primers (Table 2). The DNA fragment was further digested with *Hin*dIII/*Spe*I and ligated to the plZ1016 vector (*Hin*dIII/*Spe*I digested) by T4 ligase treatment. The pIZPHTRAP plasmid was constructed by amplifying the region 61.439-65.196 bp of plasmid 2 from *A. aromaticum* EbN1 with the 97 FW Gibson/ 95 Rv Gibson primers (Table 2). The DNA fragment was introduced into pIZPHT (*Spe*I digested) by Gibson Assembly protocol (Gibson et al. 2009. Nat Methods. 6: 343-5). The pIZPHTRAPΔDa plasmid was made from pIZPHTRAP by double digestion with SnaBI/Sphl purified without the little fragment of DNA (591 bp) belonging to o-phthaloyl-CoA decarboxylase gene. Furthermore, pIZPHTRAPΔDa SnaBI/Sphl linear plasmid was treated with Klenow enzyme and T4 ligase to recirculate it.

**Table 2**

| Primers | Sequence (5'-3')^{a} | Use |
|---|---|---|
| EbN1 113 FW | SEQ ID NO: 9. CCAAGCTTTGACCTAAGGAGGTAAATA ATGAAAAGACTGATAGTGGGGAT (HindIII) | Cloning the EB_RS21730(*phtDb*)-EB_RS21715 (*phtSa*) genes from *A. aromaticum sp*. EbN1 as a 4692 bp *HindIII*/*SpeI* DNA fragment (includes the catabolic *phtDb-phtDa-phtSb-phtSa* genes) with a synthetic Shine-Dalgarno sequence upstream of *phtDb* (double underlined) into a *HindIII*/*SpeI* double digested plZ1016 (underlined recognition site) to generate plasmid pIZPHT. |
| EbN1 102 RV | SEQ ID NO: 10. CCACTAGTTCACTGGAACGCTCCTTCG ATCTG (SpeI) | |
| 97 FW | SEQ ID NO: 11. | Cloning by Gibson |
| Gibson | GAGTATGCGGAATATCAGATCGAAGGA GCGTTCCAGTGAACAATTGACTAGTAT GTTACGAAACATCGTTTGT (MfeI-SpeI) | Assembly the *A. aromaticum* EbN1 EB_*RS21705 (phtTb)-EB_RS21700 (phtTa)* genes as a 3864 bp DNA fragment in plasmid plZPHT, to generate plasmid plZPHTRAP. |
| 95 RV Gibson | SEQ ID NO: 12. GGCGAATTGGAGCTCCACCGCGGTGG CGGCCGCTCTAGAACCTGCAGGACTA GTTCACTCCCCTCGCAGCCAGCG (XbaI-SbfI) | |
| HK *RecA* H16 Fw | SEQ ID NO: 13. AGATCGGCGTGATGTTCG | RT-PCR amplification of the *recA* gene from *C.necator* H16 |
| HK *RecA* H16 Rv | SEQ ID NO: 14. ACCGAGGCGTAGAACTTGAG | |
| *BoxB2* H16 RT Fw | SEQ ID NO: 15. GGCAGCCCAACTACCTGA | RT-PCR amplification of the *boxB₂* gene from *C. necator* H16 |
| *BoxB2* H16 RT Rv | SEQ ID NO: 16. TGCGCAGATAAATGTCATGG | |
| *BoxB1* H16 RT Fw | SEQ ID NO: 17. CGCACAGCTTCGATGTCTAC | RT-PCR amplification of the *boxB₁* gene from *C. necator* H16 |
| *BoxB 1* H16 RT Rv | SEQ ID NO: 18. GCATCTTGACGTGGTCGAA | |
| *BenA* H16 RT Fw 1 | SEQ ID NO: 19. GCCTCGACCTGGATACCTT | RT-PCR amplification of the *benA* gene from *C. necator* H16 |
| *BenA* H16 RT Rv 1 | SEQ ID NO: 20. CATCTCGAGTTCGAACAGCTC | |
| *BoxB CIB RT Fw* | SEQ ID NO: 21. AACTCGACCAACTACGACGTG | RT-PCR amplification of the *boxB* gene from *Azoarcus sp.* CIB |
| *BoxB CIB RT Rv* | SEQ ID NO: 22. GTCGTGCATCTTGACGTAGC | |

| | | |
|---|---|---|
| ^{a}Engineered restriction sites are underlined, and the corresponding restriction enzyme is shown in parenthesis. | | |

### Transport assays

Cells were grown in NB from A600 of 0.1 to A600 of 1.8≈2.0, incubated for 14h, induced by adding 1.0 mM IPTG. Cells were harvested by centrifugation, washed twice in sodium salt and suspended at a cell density of 1090 mg of protein/liter (A600 of 4.0) in sodium salt. The uptake of radioactive o-phthalate into cells was followed in a reaction mixture containing 50 µL of prewarmed cells (at 30°C for 5 min) and 50 µL of 250 µM [¹⁴C]-*o*-phthalate. Hence, assays were initiated by adding aliquots of 50 µL of cell suspensions to the vials for a final cell density of 540 mg of protein/L. The vials were incubated with shaking for 5 min at 30°C. Phthalate uptake was stopped by diluting the suspensions with 1 mL of ice-cold sodium salt, collecting the cells on a 0.22-µm-pore-size Millipore GSWP nitrocellulose filter, and washing, under suction, the cells with 5 mL of ice-cold sodium salt. The filter was dissolved in 2 ml of scintillation fluid (Cocktail Ready Safe, Beckman) and counted in an LKB Wallac liquid scintillation counter. The rate of uptake was found to be constant for greater than 10 min, and there was a linear relationship between uptake rate and cell density over a range of 270 to 2160 mg of protein/L. Where indicated 2,4-dinitrophenol (DNP) or dicyclohexylcarbodiimide (DCCD) was added to cell suspensions 10 min prior to the addition of the labelled substrate. To test the transporter's specificity the assays were performed according to the standard procedure but adding different unlabeled substrates: o-phthalate (250 mM), isophthalate (250 mM), terephthalate (250 mM).

### RNA extraction and RT-PCR assays

*Cupriavidus necator* H16 and *Azoarcus sp.* CIB cells harboring plasmid pIZPHTRAP were grown aerobically on PA-, benzoate- or pyruvate-containing MC medium until the culture reached to the end of the exponential phase. Cells were lysed in TE buffer (10 mM Tris-HCI, pH 7.5, 1 mM EDTA) containing 50 mg ml⁻¹ lysozyme. Total RNA was extracted using a High Pure RNA isolation kit (Roche), and then it was DNase-treated with a Turbo DNase kit (Ambion). The concentration and purity of the RNA samples were assessed using a Nanophotometer Pearl (Implen) according to the manufacturer's protocols, and by testing the absence of DNA through PCR amplification. Synthesis of total cDNA was performed by using the Transcriptor First Strand cDNA Synthesis kit (Roche) in 20-µl reactions containing 1 µg of RNA, 1 mM concentration of each dNTP, 10 units of reverse transcriptase, 20 units of Protector RNase Inhibitor, and 60 µM random hexamers as primers in the buffer provided by the manufacturer. The RNA and hexamers were initially heated at 65 °C for 10 min and following the addition of the rest of the components, samples were incubated at 25°C for 10 min and then at 55°C for 30 min. Reactions were terminated by incubation at 85°C for 5 min. In standard RT-PCR reactions, the cDNA was amplified with 1 unit of AmpliTaq DNA polymerase (Biotools) and 0.5 mM concentrations of the corresponding primer pairs of the *boxB* gene from *Azoarcus* sp. CIB (Table 2). Control reactions in which reverse transcriptase was omitted from the reaction mixture ensured that DNA products resulted from the amplification of cDNA rather than from DNA contamination. Real-time PCR assays were performed as described previously (Martín-Moldes et al. 2016. Proc Natl Acad Sci USA. 113:13174-9.) in a LightCycler®480 II real-time PCR system (Roche). The analysis was performed in three technical replicates from three biological samples. Reactions (20 µl) contained 1 µl of cDNA, 0.25 µM of each of the two target-specific primers, and 10 µl of SYBR Green I Master Mix (Roche). Oligonucleotides used to amplify transcripts from the *benA, boxB₁, boxB₂* and the *recA* gene of strain H16, the last one used as housekeeping, are listed in Table 2. PCR amplifications were carried out with one denaturation cycle (95 °C for 5 min), followed by 30 cycles of amplification (95 °C for 10 s, 60 °C for 10 s, and 72 °C for 10 s). After amplification, 7 melting curves were generated to confirm amplification of a single product. For relative quantification of the fluorescence values, a calibration curve was constructed by sevenfold serial dilutions of a *C. necator* H16 genomic DNA sample ranging from 1 ng to 0.5 x10-7 ng. This curve was then used as a reference standard for extrapolating the relative abundance of the cDNA target within the linear range of the curve.

### Transmission electron microscopy (TEM) studies

Bacterial cells were harvested, washed twice in saline solution and fixed in 5% (w/v) glutaraldehyde. Afterwards, cells were suspended in 2.5% (w/v) OsO₄ for 1 h, gradually dehydrated in ethanol solutions [30%, 50%, 70%, 90% and 100% (v/v); 30 min each) and propylene oxide (1h), and embedded in Epon 812 resin. Ultrathin sections were cut with a microtome using a Diatome diamond knife. The sections were picked up with 400 mesh cupper grids coated with a layer of carbon, and observed using a Jeol-1230 transmission electron microscope.

### Analysis of PHB content and monomer composition

To determine the PHB content of *Azoarcus* sp. CIB and *C. necator* H16 strains harbouring plasmid pIZPHTRAP, cells were grown at 30°C in MC medium with 3 mM benzoate or 3 mM PA under anaerobic or aerobic conditions, respectively, until they reached the stationary phase. PHB production was quantified by GC-MS of the methanolysed polyester and shown as the percentage of PHB monomer with respect to the total cell dry weight (CDW). Methanolysis procedure was carried out by suspending 5-10 mg of lyophilized cells in 0.5 ml of chloroform and 2 ml of methanol containing 15% sulfuric acid and 0.5 mg ml⁻¹ of 3-methylbenzoic acid (internal standard), and then incubated at 80°C for 7 h. After cooling, 1 ml of demineralized water and 1 ml of chloroform were added and the organic phase containing the resulting methyl esters of monomers was analyzed by GC-MS (de Eugenio et al. 2010. Environ Microbiol. 12:207-21). An Agilent series 7890A coupled with 5975C MS detector (EI, 70 eV) and a split/splitless injector were used for analysis. An aliquot (1µl) of organic phase was injected into the gas chromatograph at a split ratio 1:20. Separation of compounds was achieved using a DB-5HTDB-5HT (400 °C: 30 m ^{∗}0.25 mm ^{∗} 0.1 µm film thickness) column. Helium was used as carrier gas at a flow rate of 1 ml min⁻¹. The injector and transfer line temperature were set at 275 °C and 280 °C respectively. The oven temperature program was initial temperature 60 °C for 2 min, then from 60 °C up to 65 °C at a rate of 5 °C/min, and then from 65 °C up to 125 °C at a rate of 10 °C/min. EI mass spectra were recorded in full scan mode (m/z 40-550).

### Consumption of PA in bacterial cultures

The presence of residual PA was quantified by HPLC (Agilent Series 1260 Infinity II, Agilent, CA, USA) using on Mediterranea™ sea18 (TEKNOKROMA) (15 cm x 0,46 5µm) column, at room temperature with a flow rate of 1 mL min⁻¹ and an injection volume of 25 µl. The mobile phase was 0.1 % trifluoroacetic acid in water (A) and acetonitrile (B). The following elution program was used as follows: at the start, 100 % A; after 5 min, the percentage of B was linearly increased to 100 % in 20 min. After that, it was ramped to the original composition (100% A) in 2 min, and then equilibrated for 5 min. The retention time of PA was 13.1 min. A standard curve of PA was done (1-5 mM). As a control, the medium without PA was injected.

### Example 2 - An active PA uptake is essential for a functional pht pathway

The peripheral pathway for the anaerobic degradation of PA in denitrifying bacteria involves only two reaction steps catalyzed by the two-component SPT activating enzyme (encoded by genes *phtSa-phtSb*), and by the PCD decarboxylase (*phtDa*) and its associated UbiX-type prenyltransferase (*phtDb*) enzyme (Fig. 1A). To check if the expression of these four *pht* catabolic genes could allow growth in PA to denitrifying bacteria unable to use this aromatic compound, we cloned and expressed them in a broad-host range plasmid under the control of the *Ptac*/*lacI*^{q} regulatory couple. The resulting plasmid pIZPHT (Table 1) was then introduced into *Azoarcus sp.* CIB, an anaerobic benzoate/benzoyl-CoA degrader that lacks the pht genes and, hence, is unable to degrade PA. However, *Azoarcus sp.* CIB (pIZPHT) was unable to grow in PA as sole carbon source, even after the induction of the pht genes by the addition of IPTG to the cultures. This result suggested that the peripheral pathway for PA degradation may involve functions other than those strictly responsible for its activation/decarboxylation to benzoyl-CoA. In this sense, it is known that the aerobic degradation of phthalates requires their uptake into the bacterial cell through a specific transport system, and all the PA-induced *pht* clusters reported so far contain a couple of genes predicted to encode a TRAP transporter (Boll et al. 2020 Environmental Microbiology Reports 12(1), 3-15) (Fig. 1B). These observations suggested that an active PA uptake could be essential for the anaerobic degradation of this aromatic compound through the pht pathway.

To study PA transport in denitrifying bacteria expressing the *pht* peripheral pathway, we used *A. evansii* cells grown anaerobically in MC medium containing 5 mM benzoate or 5 mM PA. Cells suspended in in sodium salt were incubated with 25 µM [¹⁴C]-PA, and a time-course of the amount of [¹⁴C]-PA incorporated into the cells was determined. Whereas cells grown in PA showed uptake of this aromatic acid, cells grown in benzoate did not show [¹⁴C]-PA uptake (Fig. 2A). This result suggested that a specific PA transporter is induced when cells grow on this aromatic compound. To determine whether the uptake of PA requires an active transport system, *A. evansii* cells grown on PA were exposed to 2,4-dinitrophenol (DNP), an ionophore which disrupts the proton motive force, or to 1,3-dicyclohexylcarbodiimide (DCCD), which blocks ATP synthesis by acting on the ATPase complex, before the transport assay was performed. Whereas cells treated with DCCD retained more than 80% of the PA transport capacity, cells exposed to DNP lacked completely the ability to transport [¹⁴C]-PA (Fig. 2B). Thus, these results indicate that PA uptake in *A. evansii* cells requires an active transport system that is mainly dependent on the proton motive force rather than on the ATP pool, hence suggesting the involvement of an SBP-dependent secondary transporter instead of a primary ABC-type transport mechanism. Accordingly, we engineered a recombinant *pht* cassette, the genetic cassette of the invention, containing the four *pht* catabolic genes and the closely associated *phtTa-phtTb* genes likely encoding an SBP-dependent secondary transporter. The *pht* cassette was expressed in a broad-host range plasmid, the plasmid or vector of the invention, under the control of the regulatory couple *Ptac*/*lacI*^{q} generating plasmid pIZPHTRAP (Table 1, Fig. 3A). To check whether the cassette of the invention encoded a functional PA peripheral catabolic pathway, plasmid pIZPHTRAP was introduced into *Azoarcus* sp. CIB. Remarkably, *Azoarcus* sp. CIB (pIZPHTRAP) acquired the ability to use PA as sole carbon and energy source under anaerobic conditions (Fig. 3B). Therefore, this result demonstrates that both catabolic genes (*phtDa-phtDb-phtSa-phtSb*) and transport genes (*phtTa-phtTb*) are essential for the anaerobic degradation of PA via benzoyl-CoA. Taken together, we show here that an active transport system is essential for the peripheral pht pathway that metabolizes PA via CoA-derived intermediates.

### Example 3 - Characterization of the TAXI-TRAP transporter for PA uptake

We have shown above that the *phtTa-phtTb* transport genes are essential for the anaerobic degradation of PA. To confirm that the *phtTa-phtTb* genes encode a PA transporter and to determine its biochemical properties, we performed PA uptake experiments at different [¹⁴C]-PA concentrations in *Azoarcus sp.* CIB (pIZPHT), i.e., control cells that contain the catabolic pht genes but do not grow in PA, and in *Azoarcus sp.* CIB (pIZPHTRAP), i.e., cells that express the catabolic *pht* genes as well as the *phtTa-phtTb* transport genes - the genetic cassette of the invention. Whereas *Azoarcus sp.* CIB (pIZPHT) cells did not show [14C]-PA uptake, *Azoarcus* sp. CIB (pIZPHTRAP) cells showed a maximum uptake rate of about 156 ± 14 pmol min⁻¹ mg protein⁻¹, with a saturation kinetics at about 20 µM PA and a transport affinity (*Kₘ*) of 15 µM (Fig. 4A). As expected for an SBP-dependent secondary transporter, the [¹⁴C]-PA uptake was completely inhibited when cells were pre-incubated with DNP before the transport assay, but it did not decrease when cells were pre-incubated with the ATP synthase inhibitor dicyclohexylcarbodiimide (DCCD) (Fig. 4B). These results taken together confirm that *PhtTa-PhtTb* constitutes a secondary transporter responsible of PA uptake. [¹⁴C]-PA uptake rates were tested in the presence of non-radioactive phthalate isomers as competitors. As expected, when 250 µM unlabeled PA was used as competitor, the uptake rate of 25 µM [¹⁴C]-PA was completely inhibited (Fig. 4B). Interestingly, when 250 µM unlabeled isophthalate or terephthalate were used, the [¹⁴C]-PA uptake rate was reduced to 50% or 60%, respectively, of the rate in the absence of a competitor substrate (Fig. 4B). These results suggest that although the transporter has a high affinity for PA, it shows also a relatively broad substrate specificity for the other two phthalate isomers.

### Example 4 - The peripheral pht pathway allows aerobic degradation of PA in denitrifying bacteria

It is not known yet whether the *pht* pathway is strictly anaerobic *in vivo* or, on the contrary, it could allow also aerobic growth on PA when expressed under certain conditions and/or in some denitrifying strains that could metabolize benzoyl-CoA under oxic conditions. To get further insights into this issue, we checked aerobic growth of *Azoarcus* sp. CIB, that expresses a functional box pathway for the aerobic degradation of benzoyl-CoA, harboring the heterologously expressed cassette of the invention in minimal medium containing PA as sole carbon and energy source. Interestingly, *Azoarcus* sp. CIB (pIZPHTRAP) was able to grow in PA under aerobic conditions (Fig. 3C) and these PA-grown cells showed a significant induction of the endogenous box genes (Fig. 7). Therefore, this result strongly suggested that the *pht* pathway could allow the aerobic metabolism of PA via benzoyl-CoA. To further confirm such result and to discard that an endogenous enzyme expressed in aerobically-grown *Azoarcus* sp. CIB cells could functionally replace the PCD decarboxylase activity, we constructed a mutant *pht* cassette with an internal deletion of the *phtDa* gene. When the resulting plasmid pIZPHTRAP*ΔphtDa* was transferred to *Azoarcus* sp. CIB, the recombinant strain failed to grow in PA either in the presence or absence of oxygen (Figs. 3B and 3C), further suggesting that *phtDa* is essential and that no other enzyme encoded in the genome of the CIB strain can replace for this function. Taken together, the results show for the first time that the peripheral *pht* pathway may allow both the anaerobic and aerobic conversion of PA to benzoyl-CoA. In agreement with this finding, we have confirmed that *A. evansii,* which was shown to grow anaerobically on PA via the anaerobic benzoyl-CoA pathway, is also able to use PA as sole carbon and energy source in the presence of oxygen (Fig. 8). The results presented here unravel a new function for the pht pathway as an alternative peripheral route for the aerobic degradation of PA.

### Example 5 - Aerobic degradation of PA requires a functional box central pathway

As we have shown above, the peripheral *pht* pathway conferred to *Azoarcus* sp. CIB the ability to degrade PA. Under anoxic conditions, the final product of the *pht* pathway, benzoyl-CoA, is further converted to central metabolites through the anaerobic benzoyl-CoA (*bzd*) pathway. In the presence of oxygen, benzoyl-CoA is degraded through an aerobic hybrid pathway, the box pathway. To determine the catabolic requirements of the bacterial host to allow aerobic mineralization of PA via the pht peripheral pathway, we transferred plasmid pIZPHTRAP to different bacterial species unable to degrade PA and that contain different combinations of hybrid (box) and/or classical (*ben-cat*) benzoate-degradation clusters. Thus, we generated recombinant strains of *P*. *putida* KT2440, which contains only the *ben-cat* genes, *Cupriavidus pinatubonensis* JMP134, which contains a set of *ben-cat* genes and a set of *box* genes, and *C. necator* H16 (formerly known as *Ralstonia eutropha* H16), which contains a set of *ben-cat* genes in chromosome 1, a *box* cluster in chromosome 1 (*box1*), and a *box* cluster in chromosome 2 (*box2*) (Table 1).

Whereas *P*. *putida* KT2440 (pIZPHTRAP) and *C*. *pinatubonensis* JMP134 (pIZPHTRAP) grew on benzoate, they did not grow on PA. However, *C. necator* H16 (pIZPHTRAP), but not the wild-type strain lacking the pIZPHTRAP plasmid, was able to grow on PA as sole carbon source (Fig. 5A), indicating that PA was degraded via the *pht* pathway in this heterologous host. These results suggested that the classical *ben-cat* pathway was not able to metabolize the benzoyl-CoA generated by the peripheral *pht* pathway, and that only cells expressing a functional *box* pathway allowed aerobic mineralization of PA via benzoyl-CoA (Fig. 5B). In this sense, it should be mentioned that although strain JMP134 contains a *box* cluster in its genome, this cluster might not encode a functional box pathway since it lacks one of the genes (*boxD*) shown to be required for benzoyl-CoA degradation and it does not allow growth on benzoate to a JMP134ΔbenA mutant strain. To confirm that *C. necator* H16 (pIZPHTRAP) was degrading PA via the box pathway, we analyzed the expression of some marker genes of the classical *ben-cat* pathway, e.g. *benA* gene, and the hybrid box pathway, e.g., *boxB1* (for cluster *box1*) and *boxB2* (for cluster *box2)* (Fig. 5C). qRT-PCR gene expression studies of *C. necator* H16 (pIZPHTRAP) cells grown on PA were compared with those of cells grown on pyruvate (control). Whereas the basal expression of the *benA* gene was similar in pyruvate and PA, the *boxB1* and *boxB2* genes were induced in PA around 40- and 400-fold, respectively.

These results suggest that *C. necator* H16 (pIZPHTRAP) cells grown in PA cannot generate free benzoate which has been shown to be the inducer of the *ben* genes in *Cupriavidus* strains. On the contrary, the results presented strongly suggest that an efficient metabolic flux for PA degradation via the pht pathway requires the coupling to a hybrid box pathway rather than to a classical ben-cat pathway.

### Example 6 - The pht cassette as a biotechnological tool to valorize PA

Biodegradation of PA is of great interest for bioremediation strategies of PA esters that are major endocrine disrupting compounds and environmental pollutants. Moreover, PA is emerging as a very interesting feedstock generated in high amounts from the plastics additives in the up-coming plastic recycling processes. The use of PA as feedstock for the microbial synthesis of value-added biodegradable polymers, such as polyhydroxyalkanoates (PHAs), has never been demonstrated. PHAs are an environmentally friendly alternative to chemically synthesized plastics due to the fact that are bio-based, biodegradable, biocompatible, and their material properties are similar to those of petroleum-based plastics. A common type of PHAs is polyhydroxybutyrate (PHB), which contains only the 3-hydroxybutyrate monomer in its composition. Here we aimed the use of the recombinant *pht* cassette, the cassette of the invention, for the bioconversion of PA to PHB.

*C. necator* H16 is an industrially relevant microorganism generally used for its high productivity biosynthesis of PHB that can account up to 80% of the total cell dry weight. PHB production from acetyl-CoA involves the *phaAB* and *phaC* gene products (Fig. 6A) (Pohlmann et al., 2006. Nat Biotechnol.24:1257-62). Although the H16 strain has been shown to accumulate PHB granules when grown aerobically in a wide range of substrates (Tomizawa et al. 2014. ACS Sustainable Chem Eng. 2: 1106-13; Kumar et al. 2019. Biotechnol Appl Biochem. 66:153-62), it has never been tested for bioconversion of phthalates. Since we have shown above that the *pht* pathway is functional in strain H16, we tested whether *C. necator* H16 (pIZPHTRAP) cells grown aerobically on PA were able to accumulate PHB granules. Interestingly, PHB granules were observed inside the recombinant H16 cells (Fig. 6B). PHB monomer composition and cellular PHB content of lyophilized cells were determined by gas chromatography-tandem mass spectrometry (GC-MS) of the methanolysed polyester. Cells were able to accumulate PHB that accounted for 8-10% of the total cell dry weight. A similar percentage of PHB (14%) was accumulated when *C. necator* H16 (pIZPHTRAP) cells were grown in benzoate as sole carbon source. The composition of PHB was determined by GC-MS, and a single peak corresponding to the 3-hydroxybutyrate monomer was observed. Thus, all these results taken together reveal that *C. necator* H16 (pIZPHTRAP) strain constitutes a biotechnologically relevant biocatalyst to valorize PA towards the synthesis of PHB. Moreover, this invention highlights that the aerobic hybrid box pathway becomes a suitable alternative to the classical aerobic degradation pathways to funnel benzoate and benzoate-generating aromatic compounds to central metabolites, i.e. acetyl-CoA, which is then subsequently channeled through biosynthesis pathways, e.g., *pha* pathway, towards the production of PHAs, e.g., PHB (Fig. 6A).

On the other hand, we checked whether the oxygen-independent *pht* cassette allowed also PA valorization to PHB under anaerobic conditions. Since *Azoarcus* sp. CIB has been shown to accumulate PHB when degrading aromatic hydrocarbons through the anaerobic benzoyl-CoA pathway (*bzd-pim-gcd* genes) (Zamarro et al. 2017 10(6):1418-1425), we used this strain as host for testing anaerobic valorization of PA. *Azoarcus* sp. CIB (pIZPHTRAP) strain growing anaerobically on PA was shown to produce PHB, although with a 5-fold lower efficiency than that of the recombinant *C. necator* H16 strain, suggesting that these cells were able to re-route part of the 3-hydroxybutyryl-CoA generated in the anaerobic benzoyl-CoA pathway as substrate of the PhaC synthase that generates PHB (Fig. 6A).

In summary, all these results highlight the use of the genetic cassette of the invention as a biotechnological tool to engineer industrially-relevant aerobic and anaerobic PA bioconversions when expressed in suitable bacterial platforms.

### Example 7 - Conclusions

In this work we have shown that PA degradation in denitrifying bacteria via the phthaloyl-CoA peripheral pathway (*pht* pathway) requires a SBP-dependent secondary transporter (PhtTa-PhtTb) of the TAXI-TRAP class for the uptake of PA inside the bacterial cell. As far as we know this work constitutes the first in vivo experimental demonstration on the key role of an active transport system in the anaerobic catabolism of aromatic compounds. The presence of *phtTa-phtTb* genes associated to putative PA degradation clusters in bacteria belonging to different phylogenetic groups provides further insights on the functionality of the poorly characterized class of TAXI-TRAP transporters. As far as we know, this is the first report of any peripheral phthalate degradation pathway that has been successfully transferred to heterologous hosts unable to degrade phthalates. Despite the pht pathway was initially characterized as an anaerobic process for the conversion of PA to benzoyl-CoA, the results presented here expand its function as an alternative peripheral route for the aerobic degradation of PA when coupled to a functional hybrid box central pathway rather than to classical ben-cat pathways. Hence, the *pht* pathway may constitute an evolutionary acquisition for PA degradation by bacteria that drive either in anoxic environments, or in environments that face oxygen limitations and that rely on benzoyl-CoA, rather than on catecholic central intermediates, for the aerobic catabolism of aromatic compounds. The recombinant *pht* cassette developed in this work was shown to be an interesting genetic tool both to screen for functional aerobic box pathways in bacteria and to engineer industrially-relevant aerobic and anaerobic PA bioconversions when expressed in suitable bacterial platforms. Valorization of PA, an emerging feedstock from plastics recycling, towards the biosynthesis of bioplastics, e.g., PHB, in recombinant biocatalysts expressing a *pht* cassette was demonstrated for the first time. Our work also highlights that the hybrid box pathway becomes a suitable alternative to the classical aerobic degradation pathways to funnel benzoate and benzoate-generating aromatic compounds towards the production of value-added compounds such as PHAs.

## Claims

1. An isolated genetic cassette comprising:
i. an UbiX-related FMN prenyltransferase gene *(phtDb),*
ii. an UbiD-related (de) carboxylase gene (*phtDa*),
iii. a subunit B of CoA-transferase of family III gene (*phtSb*),
iv. a subunit A of CoA-transferase of family III gene (*phtSa*),
v. a periplasmic binding protein gene (*phtTb*), and
vi. a membrane-component TRAP transporter gene (*phtTa*).

2. The isolated genetic cassette according to claim 1 wherein the UbiX-related FMN prenyltransferase gene comprises SEQ ID NO: 1, the UbiD-related (de) carboxylase gene comprises SEQ ID NO: 2, the subunit B of CoA-transferase of family III gene comprises SEQ ID NO: 3, the subunit A of CoA-transferase of family III gene comprises SEQ ID NO: 4, the periplasmic binding protein gene comprises the SEQ ID NO: 5 and the membrane component TRAP transporter gene comprises SEQ ID NO: 6.

3. The isolated genetic cassette according to any of claims 1 or 2 further comprising the *p2A95* gene according to SEQ ID NO: 7.

4. The isolated genetic cassette according to any one of claims 1 to 3 further comprising the genes related to the *bzd* pathway.

5. The isolated genetic cassette according to any one of claims 1 to 3 further comprising the genes related to the *box* pathway.

6. An expression vector that comprises the isolated genetic cassette according to any one of claims 1 to 5.

7. A recombinant host cell comprising, heterologously, the isolated genetic cassette according to any one of claims 1 to 3 or the expression vector according to claim 6, wherein said expression vector comprises the isolated genetic cassette according to any one of claims 1 to 3.

8. The recombinant host cell according to claim 7, wherein the cell further comprises endogenously the genes related to the *bzd* pathway or the genes related to the *box* pathway.

9. A recombinant host cell comprising, heterologously, the isolated genetic cassette according to any of claims 4 or 5 or the expression vector according to claim 6, wherein said expression vector comprises the isolated genetic cassette according to any of claims 4 or 5.

10. The recombinant host cell according to any one of claims 7 to 9, wherein the cell is a bacterial cell.

11. The recombinant host cell according to any one of claims 7 to 10, wherein the cell belongs to the *Cupriavidus* or *Azoarcus* genus, preferably the cell belongs to the *Azoarcus* sp. CIB strain or the *Cupriavidus necator* H16 strain.

12. Use of the isolated genetic cassette according to any one of claims 1 to 5, the expression vector according to claim 6, or the recombinant host cell according to any one of claims 7 to 11 for the degradation of phthalates, preferably o-phthalate (PA).

13. The use according to claim 12, wherein the degradation is carried out aerobically.

14. Use of the isolated genetic cassette according to any of claims 4 or 5, or the expression vector according to claim 6, wherein said expression vector comprises the isolated genetic cassette according to any of claims 4 or 5, or the recombinant host cell according to any one of claims 8 to 11 for the bioconversion of PA into polyhydroxyalkanoates (PHAs), preferably polyhydroxybutyrate (PHB).

15. The use according to claim 14, wherein the bioconversion is carried out aerobically.
